# FASCICULE DE BREVET EUROPEEN

(11) **EP 1 720 837 B1**
(45) Date de publication et mention de la délivrance du brevet: **28.04.2010**
(21) Numéro de dépôt: 05717379.1
(22) Date de dépôt: 07.01.2005
(51) Int. Cl.: C07D 231/12, A61K 31/415, A61P 25/00, C07D 409/12, C07D 413/14

(54) **DERIVES DE N-'(1,5-DIPHENYL-1H-PYRAZOL-3-YL)METHYL SULFONAMIDE AVEC UNE AFFINITE POUR LES RECEPTEURS CB1**
N-'(1,5-DIPHENYL-1H-PYRAZOL-3-YL)SULFONAMIDDERIVATE MIT AFFINITÄT ZUM CB1-REZEPTOR
DERIVATIVES OF N-' (1,5-DIPHENYL-1H-PYRAZOL-3-YL) SULPHONAMIDE WITH CB1 RECEPTOR AFFINITY

(30) Priorité: 12.01.2004 FR 0400257
(43) Date de publication de la demande: 15.11.2006
(73) Titulaire: Sanofi-Aventis, 75013 Paris (FR)
(72) Inventeur: BARTH, Francis, F-34680 Saint Georges d'Orques (FR); CONGY, Christian, F-34980 Saint Gely du Fesc (FR); MARTINEZ, Serge, F-34000 Montpellier (FR); RINALDI-CARMONA, Murielle, F-34680 Saint Georges d'Orques (FR)
(74) Mandataire: Kugel, Dominique
(86) Numéro de dépôt international: PCT/FR2005/000031
(87) Numéro de publication internationale: WO 2005/073197

(56) Documents cités:
- EP-A- 0 576 357

## Description

La présente invention a pour objet des dérivés de N-[(1,5-diphényl-1*H*-pyrazol-3-yl)méthyl]sulfonamide, leur préparation et leur application en thérapeutique.

Des dérivés de diphénylpyrazole, présentant une affinité pour les récepteurs CB₁ des cannabinoïdes ont été décrits notamment dans les brevets EP 0 576 357, EP 0 656 354 et US 5 624 941.

On a maintenant trouvé des nouveaux dérivés de N-[(1,5-diphényl-1*H*-pyrazol-3-yl)méthyl]sulfonamide qui possèdent des propriétés antagonistes des récepteurs CB₁ des cannabinoïdes.

La présente invention a pour objet des composés répondant à la formule (I) : 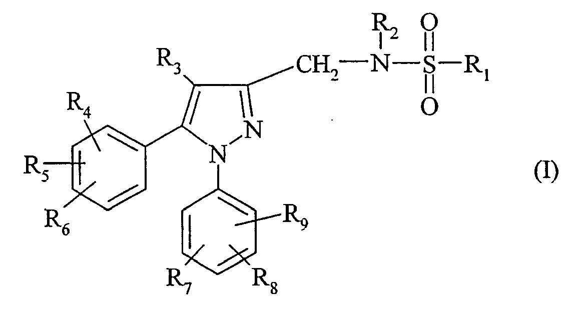 dans laquelle :
- R₁ représente
   - un (C₁-C₆)alkyle ;
   - un (C₃-C₇)cycloalkyle non substitué ou substitué une ou plusieurs fois par un groupe (C₁-C₃)alkyle ;
   - un (C₃-C₇)cycloalkylméthyle non substitué ou substitué une ou plusieurs fois sur le carbocycle par un (C₁-C₃)alkyle ;
   - un phényle non substitué ou mono-, di- ou trisubstitué par un substituant choisi indépendamment parmi un atome d'halogène, un (C₁-C₄)alkyle, un -(C₁-C₃)alcoxy, un cyano, un radical trifluorométhyle, un radical trifluorométhoxy, un groupe S(O)ₙAlk, un groupe (C₁-C₃)alkylcarbonyle, un phényle ;
   - un benzyle non substitué ou mono- ou disubstitué par un substituant choisi indépendamment parmi un atome d'halogène, un (C₁-C₃)alkyle, un (C₁-C₃)alcoxy ; un radical trifluorométhyle ;
   - un thiényle non substitué ou substitué par un atome d'halogène ou par un isoxazolyle ;
- R₂ représente un atome d'hydrogène ou un (C₁-C₃)alkyle ;
- R₃ représente un atome d'hydrogène ou un (C₁-C₅)alkyle ;
- R₄, R₅, R₆, R₇, R₈ et R₉ représentent chacun indépendamment un atome d'hydrogène, un atome d'halogène, un (C₁-C₇)alkyle, un (C₁-C₅)alcoxy, un radical trifluorométhyle ou un groupe S(O)ₙAlk ;
- n représente 0, 1 ou 2 ;
- Alk représente un (C ₁-C₄)alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₃)alkyle ou respectivement (C₁-C₄)alkyle, (C₁-C₅)alkyle, (C₁-C₆)alkyle ou (C₁-C₇)alkyle, on entend un radical alkyle linéaire ou ramifié de un à trois atomes de carbone ou respectivement de un à quatre atomes de carbone, de un à cinq atomes de carbone, de un à six atomes de carbone ou de un à sept atomes de carbone, tel que le radical méthyle, éthyle, propyle, isopropyle, butyle, isobutyle, *sec-*butyle, *tert*-butyle, pentyle, isopentyle, hexyle, isohexyle, heptyle.

Par (C₁-C₃)alcoxy ou respectivement (C₁-C₅)alcoxy, on entend un radical alcoxy linéaire ou ramifié de un à trois atomes de carbone ou respectivement de un à cinq atomes de carbone, tel que le radical méthoxy, éthoxy, propoxy, isopropoxy, butoxy, *sec*-butoxy, *tert*-butoxy, pentoxy, isopentoxy.

Par (C₃-C₇)cycloalkyle on entend un groupe alkyle cyclique de 3 à 7 atomes de carbone, tel que le groupe cyclopropyle, cyclobutyle, cyclopentyle, cyclohexyle, cycloheptyle.

Parmi les composés de formule (I), objets de l'invention, on peut citer les composés préférés qui se définissent comme suit :
- R₁ représente
   . un éthyle, un isopropyle, un n-butyle ;
   . un cyclohexyle ;
   . un cyclohexylméthyle ;
   . un 2-chlorophényle, un 3-chlorophényle, un 2-fluorophényle, un 3-chloro-4-fluorophényle, un 4-bromo-2-éthylphényle, un 3-méthylphényle, un 4-*tert-*butylphényle, un 3,5-diméthylphényle, un 3-méthoxyphényle, un 4-méthoxyphényle, un 3-cyanophényle, un 4-cyanophényle, un 2-(trifluorométhyl)phényle, un 3-(trifluorométhyl)phényle, un 4-(trifluorométhyl)phényle, un 3,5-bis(trifluorométhyl)phényle, un 2-(trifluorométhoxy)phényle, un 3-(trifluorométhoxy)phényle, un 2-(méthylsulfonyl)phényle, un 3-(méthylsulfonyl)phényle, un 3-acétylphényle, un 3-biphényle, un 2-biphényle ;
   . un 3-chlorobenzyle, un 2-fluorobenzyle, un 4-fluorobenzyle, un 3-(trifluorométhyl)benzyle, un 4-(trifluorométhyl)benzyle ;
   . un 5-bromo-2-thiényle ; un 5-isoxazol-3-yl-2-thiényle ;
- et/ou R₂ représente un atome d'hydrogène ou un méthyle ;
- et/ou R₃ représente un méthyle ou un éthyle ;
- et/ou R₄ représente un atome d'hydrogène ;
- et/ou R₅ est en position -4- du phényle et représente un atome de brome, de chlore, de fluor ou un méthoxy ;
- et/ou R₆ représente un atome d'hydrogène ;
- et/ou R₇ représente un atome d'hydrogène ;
- et/ou R₈ est en position -4- du phényle et représente un atome d'hydrogène, un atome de chlore, un atome de fluor ;
- et/ou R₉ est en position -2- du phényle et représente un atome de chlore ou de fluor ;
à l'état de base ou de sel d'addition à un acide, ainsi qu'à l'état d'hydrate ou de solvat.

Parmi ces derniers composés préférés, on préfère particulièrement, les composés de formule (I) pour lesquels :
- R₁ représente
   . un éthyle, un isopropyle, un n-butyle ;
   . un cyclohexyle ;
   . un cyclohexylméthyle ;
   . un 2-chlorophényle, un 3-chlorophényle, un 2-fluorophényle, un 3-chloro-4-fluorophényle, un 4-bromo-2-éthylphényle, un 3-méthylphényle, un 4-*tert-*butylphényle, un 3,5-diméthylphényle, un 3-méthoxyphényle, un 4-méthoxyphényle, un 3-cyanophényle, un 4-cyanophényle, un 2-(trifluorométhyl)phényle, un 3-(trifluorométhyl)phényle, un 4-(trifluorométhyl)phényle, un 3,5-bis(trifluorométhyl)phényle, un 2-trifluorométhoxy)phényle, un 3-(trifluorométhoxy)phényle, un 2-(méthylsulfonyl)phényle, un 3-(méthylsulfonyl)phényle, un 3-acétylphényle, un 3-biphényle, un 2-biphényle ;
   . un 3-chlorobenzyle, un 2-fluorobenzyle, un 4-fluorobenzyle, un 3-(trifluorométhyl)benzyle, un 4-(trifluorométhyl)benzyle ;
   . un 5-bromo-2-thiényle ; un 5-isoxazol-3-yl-2-thiényle ;
- R₂ représente un atome d'hydrogène ou un méthyle ;
- R₃ représente un méthyle ou un éthyle ;
- R₄ représente un atome d'hydrogène ;
- R₅ est en position -4- du phényle et représente un atome de brome, de chlore, de fluor ou un méthoxy ;
- R₆ représente un atome d'hydrogène ;
- R₇ représente un atome d'hydrogène ;
- R₈ est en position -4- du phényle et représente un atome d'hydrogène, un atome de chlore, un atome de fluor ;
- R₉ est en position -2- du phényle et représente un atome de chlore ou de fluor ;
à l'état de base ou de sel d'addition à un acide, ainsi qu'à l'état d'hydrate ou de solvat.

Parmi les composés de formule (I) objets de l'invention, on peut notamment citer les composés suivants :
- N-[[5-(4-bromophényl)-1-(2,4-dichlorophényl)-4-méthyl-1*H*-pyrazol-3-yl]méthyl]butane-1-sulfonamide ;
- N-[[5-(4-bromophényl)-1-(2,4-dichlorophényl)-4-méthyl-1*H*-pyrazol-3-yl]méthyl]cyclohexanesulfonamide ;
- N-[[5-(4-bromophényl)-1-(2,4-dichlorophényl)-4-méthyl-1*H*-pyrazol-3-yl]méthyl]cyclohexylméthanesulfonamide ;
- N-[[5-(4-bromophényl)-1-(2,4-dichlorophényl)-4-méthyl-1*H*-pyrazol-3-yl]méthyl]-3-chlorobenzènesulfonamide ;
- N-[[5-(4-bromophényl)-1-(2,4-dichlorophényl)-4-méthyl-1*H*-pyrazol-3-yl]méthyl]-4-*tert*-butylbenzènesulfonamide ;
- N-[[5-(4-bromophényl)-1-(2,4-dichlorophényl)-4-méthyl-1*H*-pyrazol-3-yl]méthyl]-3-méthoxybenzènesulfonamide ;
- N-[[5-(4-bromophényl)-1-(2,4-dichlorophényl)-4-méthyl-1*H*-pyrazol-3-yl]méthyl]-4-méthoxybenzènesulfonamide ;
- N-[[5-(4-bromophényl)-1-(2,4-dichlorophényl)-4-méthyl-1*H*-pyrazol-3-yl]méthyl]-4-(trifluorométhyl)benzènesulfonamide ;
- N-[[5-(4-bromophényl)-1-(2,4-dichlorophényl)-4-méthyl-1*H*-pyrazol-3-yl]méthyl]-2-(méthylsulfonyl)benzènesulfonamide ;
- N-[[5-(4-bromophényl)-1-(2,4-dichlorophényl)-4-méthyl-1*H*-pyrazol-3-yl]méthyl]-1-(3-chlorophényl)méthanesulfonamide ;
- N-[[5-(4-bromophényl)-1-(2,4-dichlorophényl)-4-méthyl-1*H*-pyrazol-3-yl]méthyl]-1-[3-(trifluorométhyl)phényl]méthanesulfonamide ;
- N-[[5-(4-bromophényl)-1-(2,4-dichlorophényl)-4-méthyl-1*H*-pyrazol-3-yl]méthyl]-3-chloro-4-fluorobenzènesulfonamide ;
- N-[[5-(4-chlorophényl)-1-(2,4-dichlorophényl)-4-méthyl-1*H*-pyrazol-3-yl]méthyl]-butane-1-sulfonamide ;
- 3-chloro-N-[[5-(4-chlorophényl)-1-(2,4-dichlorophényl)-4-méthyl-1*H*-pyrazol-3-yl]méthyl]benzènesulfonamide ;
- 4-*tert*-butyl-N-[[5-(4-chlorophényl)-1-(2,4-dichlorophényl)-4-méthyl-1*H*-pyrazol-3-yl]méthyl]benzènesulfonamide ;
- N-[[5-(4-chlorophényl)-1-(2,4-dichlorophényl)-4-méthyl-1*H-*pyrazol-3-yl]méthyl]-3-méthoxybenzènesulfonamide ;
- N-[[5-(4-chlorophényl)-1-(2,4-dichlorophényl)-4-méthyl-1*H*-pyrazol-3-yl]méthyl]-3-cyanobenzènesulfonamide ;
- N-[[5-(4-chlorophényl)-1-(2,4-dichlorophényl)-4-méthyl-1*H*-pyrazol-3-yl]méthyl]-4-(trifluorométhyl)benzènesulfonamide ;
- N-[[5-(4-chlorophényl)-1-(2,4-dichlorophényl)-4-méthyl-1*H*-pyrazol-3-yl]méthyl]-2-(trifluorométhoxy)benzènesulfonamide ;
- N-[[5-(4-chlorophényl)-1-(2,4-dichlorophényl)-4-méthyl-1*H*-pyrazol-3-yl]méthyl]-2-(méthylsulfonyl)benzènesulfonamide ;
- 3-chloro-N-[[5-(4-chlorophényl)-1-(2,4-dichlorophényl)-4-méthyl-1*H*-pyrazol-3-yl]méthyl]-4-fluorobenzènesulfonamide ;
- 4-bromo-N-[[5-(4-chlorophényl)-1-(2,4-dichlorophényl)-4-méthyl-1*H*-pyrazol-3-yl]méthyl]-2-éthylbenzènesulfonamide ;
- N-[[5-(4-bromophényl)-1-(2,4-dichlorophényl)-4-éthyl-1*H*-pyrazol-3-yl]méthyl] éthanesulfonamide ;
- N-[[5-(4-bromophényl)-1-(2,4-dichlorophényl)-4-éthyl-1*H*-pyrazol-3-yl]méthyl] propane-2-sulfonamide ;
- N-[[5-(4-bromophényl)-1-(2,4-dichlorophényl)-4-éthyl-1*H-*pyrazol-3-yl]méthyl] butane-1-sulfonamide ;
- N-[[5-(4-bromophényl)-1-(2,4-dichlorophényl)-4-éthyl-1*H*-pyrazol-3-yl]méthyl] cyclohexanesulfonamide ;
- N-[[5-(4-bromophényl)-1-(2,4-dichlorophényl)-4-éthyl-1*H*-pyrazol-3-yl]méthyl]-1-cyclohexylméthanesulfonamide ;
- N-[[5-(4-bromophényl)-1-(2,4-dichlorophényl)-4-éthyl-1*H*-pyrazol-3-yl]méthyl]-3-chlorobenzènesulfonamide ;
- N-[[5-(4-bromophényl)-1-(2,4-dichlorophényl)-4-éthyl-1*H*-pyrazol-3-yl]méthyl]-2-chlorobenzènesulfonamide ;
- N-[[5-(4-bromophényl)-1-(2,4-dichlorophényl)-4-éthyl-1*H*-pyrazol-3-yl]méthyl]-3-méthylbenzènesulfonamide ;
- N-[[5-(4-bromophényl)-1-(2,4-dichlorophényl)-4-éthyl-1*H*-pyrazol-3-yl]méthyl]-4-*tert*-butylbenzènesulfonamide ;
- N-[[5-(4-bromophényl)-1-(2,4-dichlorophényl)-4-éthyl-1*H*-pyrazol-3-yl]méthyl]-4-méthoxybenzènesulfonamide ;
- N-[[5-(4-bromophényl)-1-(2,4-dichlorophényl)-4-éthyl-1*H*-pyrazol-3-yl]méthyl]-3-méthoxybenzènesulfonamide ;
- N-[[5-(4-bromophényl)-1-(2,4-dichlorophényl)-4-éthyl-1*H*-pyrazol-3-yl]méthyl]-4-(trifluorométhyl)benzènesulfonamide ;
- N-[[5-(4-bromophényl)-1-(2,4-dichlorophényl)-4-éthyl-1*H*-pyrazol-3-yl]méthyl]-3-(trifluorométhyl)benzènesulfonamide ;
- N-[[5-(4-bromophényl)-1-(2,4-dichlorophényl)-4-éthyl-1*H*-pyrazol-3-yl]méthyl]-2-(trifluorométhyl)benzènesulfonamide ;
- N-[[5-(4-bromophényl)-1-(2,4-dichlorophényl)-4-éthyl-1*H*-pyrazol-3-yl]méthyl]-3-(trifluorométhoxy)benzènesulfonamide ;
- N-[[5-(4-bromophényl)-1-(2,4-dichlorophényl)-4-éthyl-1*H*-pyrazol-3-yl]méthyl]-2-(trifluorométhoxy)benzènesulfonamide ;
- 3-acétyl-N-[[5-(4-bromophényl)-1-(2,4-dichlorophényl)-4-éthyl-1*H*-pyrazol-3-yl]méthyl]benzènesulfonamide ;
- N-[[5-(4-bromophényl)-1-(2,4-dichlorophényl)-4-éthyl-1*H*-pyrazol-3-yl]méthyl] biphényl-3-sulfonamide ;
- N-[[5-(4-bromophényl)-1-(2,4-dichlorophényl)-4-éthyl-1*H*-pyrazol-3-yl]méthyl]-1-[4-(trifluorométhyl)phényl]méthanesulfonamide ;
- N-[[5-(4-bromophényl)-1-(2,4-dichlorophényl)-4-éthyl-1*H*-pyrazol-3-yl]méthyl]-1-[3-(trifluorométhyl)phényl]méthanesulfonamide ;
- N-[[5-(4-bromophényl)-1-(2,4-dichlorophényl)-4-éthyl-1*H*-pyrazol-3-yl]méthyl]-3,5-diméthylbenzènesulfonamide ;
- N-[[5-(4-bromophényl)-1-(2,4-dichlorophényl)-4-éthyl-1*H*-pyrazol-3-yl]méthyl]-3,5-bis(trifluorométhyl)benzènesulfonamide ;
- 3-chloro-N-[[1-(2-chlorophényl)-5-(4-chlorophényl)-4-méthyl-1*H*-pyrazol-3-yl]méthyl]benzènesulfonamide ;
- N-[[1-(2-chlorophényl)-5-(4-chlorophényl)-4-méthyl-1*H*-pyrazol-3-yl]méthyl]-2-fluorobenzènesulfonamide ;
- N-[[1-(2-chlorophényl)-5-(4-chlorophényl)-4-méthyl-1*H*-pyrazol-3-yl]méthyl]-3-cyanobenzènesulfonamide ;
- N-[[1-(2-chlorophényl)-5-(4-chlorophényl)-4-méthyl-1*H*-pyrazol-3-yl]méthyl]-3-méthoxybenzènesulfonamide ;
- N-[[5-(4-bromophényl)-1-(2,4-dichlorophényl)-4-méthyl-1*H*-pyrazol-3-yl]méthyl]-3-méthoxybenzènesulfonamide ;
- N-[[5-(4-bromophényl)-1-(2,4-dichlorophényl)-4-méthyl-1*H*-pyrazol-3-yl]méthyl]-3-cyanobenzènesulfonamide ;
- N-[[5-(4-bromophényl)-1-(2,4-dichlorophényl)-4-éthyl-1*H*-pyrazol-3-yl]méthyl]-1-(2-fluorophényl)méthanesulfonamide ;
- N-[[5-(4-bromophényl)-1-(2,4-dichlorophényl)-4-éthyl-1*H*-pyrazol-3-yl]méthyl]-1-(4-fluorophényl)méthanesulfonamide ;
- 5-bromo-N-[[5-(4-bromophényl)-1-(2,4-dichlorophényl)-4-éthyl-1*H*-pyrazol-3-yl]méthyl]thiophène-2-sulfonamide ;
- N-[[5-(4-bromophényl)-1-(2,4-dichlorophényl)-4-éthyl-1*H*-pyrazol-3-yl]méthyl]-5-isoxazol-3-ylthiophène-2-sulfonamide ;
- 3-chloro-N-[[1-(2,4-dichlorophényl)-5-(4-méthoxyphényl)-4-méthyl-1*H*-pyrazol-3-yl]méthyl]benzènesulfonamide ;
- N-[[1-(2,4-dichlorophényl)-5-(4-méthoxyphényl)-4-méthyl-1*H*-pyrazol-3-yl]méthyl]-3-méthylbenzènesulfonamide ;
à l'état de base ou de sel d'addition à un acide, ainsi qu'à l'état d'hydrate ou de solvat.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Group in Organic Synthesis", Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advances in Organic Chemistry", J. March, 3rd Edition, Wiley Interscience, 1985, p. 310-316.

Conformément à l'invention, on peut préparer les composés de formule (I) selon un procédé qui est **caractérisé en ce que** : on fait réagir, en présence d'une base et dans un solvant, un composé de formule : 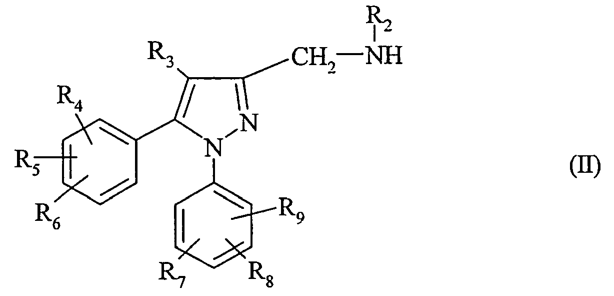 dans laquelle R₂, R₃, R₄, R₅, R₆, R₇, R₈ et R₉ sont tels que définis pour un composé de formule (I), avec un halogénure de sulfonyle de formule : 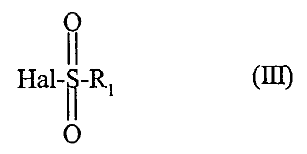 dans laquelle R₁ est tel que défini pour un composé de formule (I) et Hal représente un atome d'halogène.

Eventuellement, on transforme le composé de formule (I) en l'un de ses sels d'addition à un acide.

La réaction s'effectue en présence d'une base telle que la triéthylamine ou la diisopropyléthylamine, dans un solvant tel que le dichlorométhane ou le tétrahydrofurane, et à une température comprise entre la température ambiante et la température de reflux du solvant. La réaction s'effectue de préférence en utilisant un composé de formule (III) dans laquelle Hal représente un atome de chlore.

Selon une autre variante du procédé on peut préparer un composé de formule (I) dans laquelle R₂ représente un (C₁-C₃)alkyle par réaction d'un composé de formule (I) dans laquelle R₂ = H avec un halogénure de (C₁-C₃)alkyle, en présence d'une base telle que l'hydrure de sodium, ou le carbonate de potassium, dans un solvant tel que le N,N-diméthylformamide et à une température comprise entre la température ambiante et la température de reflux du solvant.

Les composés de formule (I) ainsi obtenus peuvent être ultérieurement séparés du milieu réactionnel et purifiés selon les méthodes classiques, par exemple par cristallisation ou chromatographie.

Les composés de formule (II) se préparent par réaction d'un composé de formule : 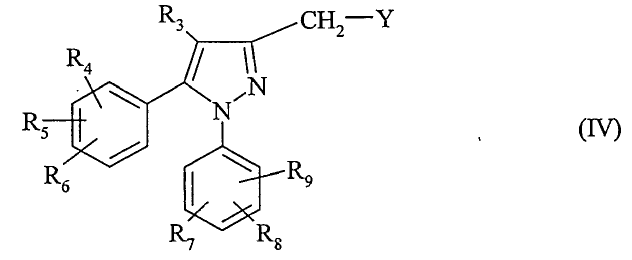 dans laquelle R₃, R₄, R₅, R₆, R₇, R₈ et R₉ sont tels que définis pour un composé de formule (I) et Y représente un groupe partant tel que défmi ci-dessus, de préférence un atome d'halogène ou un groupe hydroxy activé tel qu'un groupe méthanesulfonate, benzène sulfonate, p-toluènesulfonate ou triflate, avec un composé de formule :

H₂N-R₂ (V)

dans laquelle R₂ est tel que défini pour un composé de formule (I).

La réaction s'effectue dans un solvant tel que N,N-diméthylformamide, l'acétonitrile, le dichlorométhane, le toluène ou le propan-2-ol, et en présence ou en l'absence d'une base. Lorsqu'on utilise une base, celle-ci est choisie parmi les bases organiques telles que la triéthylamine, la N,N-diisopropyléthylamine ou la N-méthylmorpholine. La réaction s'effectue à une température comprise entre 0°C et la température de reflux du solvant.

Selon une variante, on peut également préparer un composé de formule (II) dans laquelle R₂ = H par réaction d'un composé de formule (IV) dans laquelle Y = Cl avec le 1,3,5,7-tétraazatricyclo[3.3.1^{3,7}]décane (ou hexaméthylènetétramine) suivi d'une hydrolyse par un acide fort tel que l'acide chlorhydrique.

Les composés de formule (III) sont disponibles dans le commerce ou décrits dans la littérature, ou peuvent être préparés selon des méthodes qui y sont décrites telles que dans J. Org. Chem. USSR, 1970, 6, 2454-2458 ; J. Am. Chem. Soc., 1952, 74, 2008 ; J. Med.Chem., 1977, 20(10), 1235-1239 ; EP0469 984 ; WO95/18105.

Par exemple les composés de formule (III) peuvent être préparés par halogénation des acides sulfoniques correspondants ou de leurs sels, par exemple de leur sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant tel qu'un hydrocarbure halogéné ou le N,N-diméthylformamide et à une température comprise entre -10°C et 200°C.

Les composés de formule (IV) se préparent à partir des composés de formule : 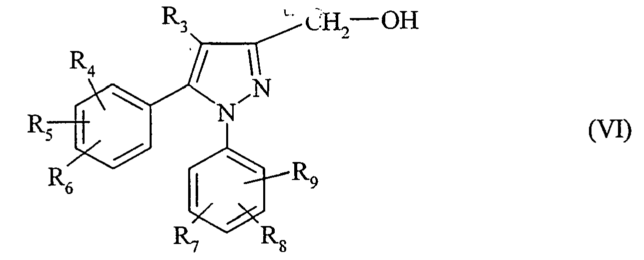 dans laquelle R₃, R₄, R₅, R₆, R₇, R₈ et R₉ sont tels que définis pour un composé de formule (I), selon des méthodes classiques précédemment citées.

Ainsi, par exemple, lorsque dans un composé de formule (IV) Y représente un atome d'halogène, on traite un composé de formule (VI) par un agent d'halogénation tel que PCl₅, PBr₃, HBr ou BBr₃, dans un solvant tel que le dichlorométhane et à une température comprise entre 0°C et la température ambiante.

Lorsque, dans un composé de formule (IV), Y représente un méthanesulfonate, un benzènesulfonate, un p-toluènesulfonate ou un trifluorométhanesulfonate, on fait réagir un composé de formule (VI) avec un chlorure de sulfonyle de formule X-SO₂-Cl dans laquelle X représente un méthyle, un phényle, un p-tolyle ou un trifluorométhyle. La réaction s'effectue en présence d'une base telle que la triéthylamine, la pyridine ou la N,N-diisopropyléthylamine, dans un solvant tel que le dichlorométhane ou le toluène et à une température comprise entre -20°C et la température de reflux du solvant.

Les composés de formule (V) sont connus.

Les composés de formule (VI) se préparent par une réaction de réduction des composés de formule : 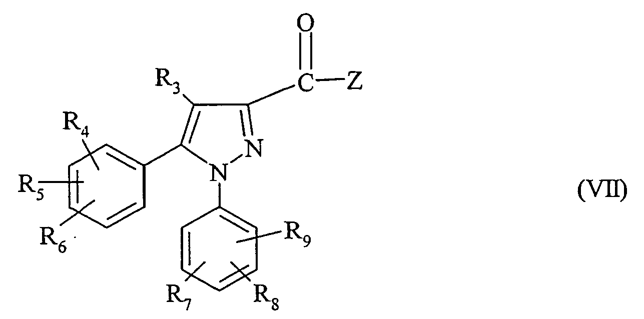 dans laquelle R₃, R₄, R₅, R₆, R₇, R₈ et R₉ sont tels que définis pour un composé de formule (I) et Z représente un hydroxy ou un (C₁-C₂)alcoxy.

La réaction s'effectue en présence d'un agent réducteur tel que le borohydrure de sodium ou l'hydrure d'aluminium et de lithium, dans un solvant tel que le tétrahydrofurane, et à une température comprise entre -20°C et la température ambiante. Lorsqu'on réduit un composé de formule (VII) dans laquelle Z = OH, l'acide peut être précédemment activé par réaction avec du chloroformiate d'éthyle en présence de triéthylamine.

Les composés de formule (VII) sont connus et se préparent selon des méthodes connues telles que celles décrites dans EP 0656 354, EP 0576 357 ou dans WO 00/46209.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU V ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DIPEA : diisopropyléthylamine
TFA : acide trifluoroacétique
Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de KHSO₄ et 32,32 g de K₂SO₄ dans 1 litre d'eau.

Les spectres de résonance magnétique nucléaire sont enregistrés à 200 MHz dans le DMSO-d₆. Pour l'interprétation des spectres, on utilise les abréviations suivantes : s : singulet, d : doublet, t : triplet, m : massif, mt : multiplet, se : singulet élargi.

Les composés selon l'invention sont analysés par couplage LC/UV/MS (chromatographie liquide/détection UV/spectrométrie de masse). On mesure le pic moléculaire (MH⁺) et le temps de rétention (tr) en minutes.

L'appareil utilisé, commercialisé par Agilent, est composé d'un chromatographe HP 1100 équipé d'un détecteur à barette de diodes Agilent et d'un spectromètre de masse quadripolaire MSD Quad.

### Méthode A :

On utilise une colonne Xterra Waters^{®} MS C18, commercialisée par Waters, de 2,1 x 30 mm, 3,5 µm, à température ambiante, débit 1 ml/minute.

L'éluant est composé comme suit :
- solvant A : 0,025 % d'acide trifluoroacétique (TFA) dans l'eau ;
- solvant B : 0,025 % de TFA dans l'acétonitrile.

Gradient : Le pourcentage de solvant B varie de 0 à 100 % en 2 minutes avec un plateau à 100 % de B pendant 1 minute.

La détection UV est effectuée entre 210 nm et 400 nm et la détection de masse en mode ionisation chimique à pression atmosphérique.

### Méthode B :

On utilise une colonne Symmetry C 18 de 2,1 x 50 mm, 3,5 µm, à 30°C, débit 0,4 ml/minute.

L'éluant est composé comme suit :
- solvant A : 0,005 % d'acide trifluoroacétique (TFA) dans l'eau à pH 3,15 ;
- solvant B : 0,005 % de TFA dans l'acétonitrile.

**Gradient :**

| Temps (minute) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à λ = 210 nM et la détection de masse est effectuée en mode électrospray (ESI) positif.

### Méthode C :

On utilise une colonne Symmetry C 18 de 2,1 x 50 mm, 3,5 µm, à 30°C, débit 0,4 ml/minute.

L'éluant est composé comme suit :
- solvant A : 0,005 % de TFA dans l'eau à pH 3,15 ;
- solvant B : 0,005 % de TFA dans l'acétonitrile.

**Gradient :**

| Temps (minute) | % A | % B |
|---|---|---|
| 0 | 100 | 0 |
| 20 | 10 | 90 |
| 30 | 10 | 90 |
| 35 | 100 | 0 |
| 40 | 100 | 0 |

La détection UV est effectuée à λ = 210 nM et la détection de masse est effectuée en mode électrospray (ESI) positif.

### Méthode D :

On utilise une colonne Xterra MS C 18 de 2,1 x 50 mm, 3,5 µm, à 30°C, débit 0,4 ml/minute.

L'éluant est composé comme suit :
- solvant A : acétate d'ammonium (AcONH₄) 10 nM dans l'eau à pH 7 ;
- solvant B : acétonitrile.

**Gradient :**

| Temps (minute) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à λ = 220 nM et la détection de masse est effectuée en mode électrospray (ESI) positif.

### Méthode E :

On utilise une colonne Xterra MS C 18 de 2,1 x 50 mm, 3,5 µm, à 30°C, débit 0,4 ml/minute.

L'éluant est composé comme suit :
- solvant A : AcONH₄ 10 nM dans l'eau à pH 7 ;
- solvant B : acétonitrile.

**Gradient :**

| Temps (minute) | % A | % B |
|---|---|---|
| 0 | 100 | 0 |
| 20 | 10 | 90 |
| 30 | 10 | 90 |
| 35 | 100 | 0 |
| 40 | 100 | 0 |

### PREPARATIONS

### 1. Préparations des composés de formule (VII).

### Préparation 1.1

### Acide 5-(4-bromophényl)-1-(2,4-dichlorophényl)-4-méthyl-1H-pyrazole-3-carboxylique.

(VII) : R₃ = -CH₃ ; R₄ = H ; R₅ = 4-Br ; R₆ = H ; R₇ = H ; R₈ = 4-Cl ; R₉ = 2-Cl; Z = -OH.

### A) Sel de lithium du 4-(4-bromophényl)-3-méthyl-2-oxo-4-oxydobut-3-énoate d'éthyle.

On refroidit à -60°C une solution de 43 g du sel de lithium de l'hexaméthyldisilazane dans 300 ml d'éther, ajoute, goutte à goutte, une solution de 50 g de 4-bromopropiophénone dans 500 ml d'éther et laisse sous agitation jusqu'à ce que la température remonte à -30°C. On ajoute ensuite 38 g d'oxalate de diéthyle et laisse 18 heures sous agitation en laissant remonter la température à TA. On essore le précipité formé, le lave à l'éther et le sèche sous vide. On obtient 62 g du produit attendu.

### B) 5-(4-Bromophényl)-1-(2,4-dichlorophényl)-4-méthyl-1H-pyrazole-3-carboxylate d'éthyle.

A une solution de 30 g du composé obtenu à l'étape précédente dans 150 ml d'acide acétique on ajoute 20 g de chlorhydrate de 2,4-dichlorophénylhydrazine et chauffe à reflux pendant 3 heures. Après refroidissement à TA, on verse le mélange réactionnel dans un mélange eau/glace, extrait à l'éther, lave la phase organique par une solution saturée de NaCl, sèche sur MgSO₄, évapore partiellement le solvant sous vide et essore le produit cristallisé formé. On obtient 33,4 g du produit attendu.

### C) Acide 5-(4-bromophényl)-1-(2,4-dichlorophényl)-4-méthyl-1H-pyrazole-3-carboxylique.

A une solution de 26 g du composé obtenu à l'étape précédente dans 50 ml d'EtOH on ajoute 6,5 g de KOH puis 20 ml d'eau et chauffe à reflux pendant 2 heures. Après refroidissement à TA, on verse le mélange réactionnel dans un mélange eau/glace contenant 10 ml d'HCl concentré, essore le précipité formé, le lave à l'eau et le sèche sous vide. On obtient 24 g du produit attendu.

### Préparation 1.2

### 5-(4-Chlorophényl)-1-(2,4-dichlorophényl)-4-méthyl-1H-pyrazole-3-carboxylate de méthyle.

(VII) : R₃ = -CH₃ ; R₄ = H ; R₅ = 4-Cl ; R₆ = H ; R₇ = H ; R₈ = 4-Cl ; R₉ = 2-Cl; Z = -OCH₃.

### A) Acide 5-(4-chlorophényl)-1-(2,4-dichlorophényl)-4-méthyl-1H-pyrazole-3-carboxylique.

On prépare ce composé selon les méthodes opératoires décrites dans EP 0 656 354B.

### B) 5-(4-Chlorophényl)-1-(2,4-dichlorophényl)-4-méthyl-1H-pyrazole-3-carboxylate de méthyle.

A une solution de 30 g du composé obtenu à l'étape précédente dans 500 ml de MeOH, on ajoute 3 g d'acide paratoluènesulfonique et chauffe à reflux pendant 2 heures. On concentre sous vide de moitié le mélange réactionnel, essore le précipité formé, le lave au MeOH et le sèche. On obtient 30 g du produit attendu.

### Préparation 1.3

### Acide 5-(4-bromophényl)-1-(2,4-dichlorophényl)-4-éthyl-1H-pyrazole-3-carboxylique.

(VII) : R₃ = -CH₂CH₃ ; R₄ = H ; R₅ = 4-Br ; R₆ = H ; R₇ = H ; R₈ = 4-Cl ; R₉ = 2-Cl; Z = -OH.

On prépare ce composé selon les modes opératoires décrits dans WO 00/46209.

En suivant les modes opératoires décrits dans les Préparations 1 ci-dessus, on prépare les composés de formule (VII) rassemblés dans le TABLEAU I ci-après.

**TABLEAU I**

| | | | | | |
|---|---|---|---|---|---|
| 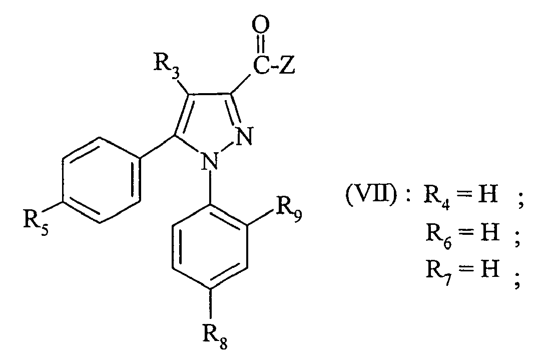 | | | | | |

| Préparations | R₃ | R₅ | R₈ | R₉ | Z |
|---|---|---|---|---|---|
| 1.1 | -CH₃ | Br | Cl | Cl | -OH |
| 1.2 | -CH₃ | Cl | Cl | Cl | -OCH₃ |
| 1.3 | -CH₂CH₃ | Br | Cl | Cl | -OH |
| 1.4 | -CH₃ | Cl | H | Cl | -OCH₂CH₃ |
| 1.5 | -CH₃ | F | H | Cl | -OCH₂CH₃ |
| 1.6 | -CH₃ | -OCH₃ | H | Cl | -OCH₂CH₃ |
| 1.7 | -CH₃ | -OCH₃ | H | F | -OCH₂CH₃ |
| 1.8 | -CH₃ | -OCH₃ | Cl | Cl | -OCH₂CH₃ |
| 1.9 | -CH₃ | -OCH₃ | F | F | -OCH₂CH₃ |

### 2. Préparations des composés de formule (VI).

### Préparation 2.1

### [5-(4-Bromophényl)-1-(2,4-dichlorophényl)-4-méthyl-1H-pyrazol-3-yl]méthanol.

(VI) : R₃ = -CH₃ ; R₄ = H ; R₅ = 4-Br ; R₆ = H ; R₇ = H ; R₈ = 4-Cl ; R₉ = 2-Cl.

On refroidit à -10°C un mélange de 24 g du composé obtenu à la Préparation 1.1 dans 200 ml de THF, ajoute 7,8 ml de triéthylamine puis, goutte à goutte, 5,38 ml de chloroformiate d'éthyle et laisse 15 minutes sous agitation à -10°C. On ajoute ensuite, en une seule fois et à une température inférieure à -10°C, 6,3 g de borohydrure de sodium puis, goutte à goutte, 100 ml de MeOH et laisse 30 minutes sous agitation à 0°C. On hydrolyse le mélange réactionnel par ajout de 100 ml d'HCl à 10 %, concentre sous vide, reprend le résidu à l'eau, extrait à l'AcOEt, lave la phase organique par une solution saturée de NaCl, sèche sur MgSO₄ et évapore le solvant sous vide. On chromatographie le résidu sur gel de silice en éluant par le mélange cyclohexane/AcOEt (75/25 ; v/v). On obtient 22 g du produit attendu.

### Préparation 2.2

### [5-(4-Chlorophényl)-1-(2,4-dichlorophényl)-4-méthyl-1H-pyrazol-3-yl]méthanol.

(VI) : R₃ = -CH₃ ; R₄ = H ; R₅ = 4-Cl ; R₆ = H ; R₇ = H ; R₈ = 4-Cl ; R₉ = 2-Cl.

On refroidit à -5°C une solution de 30 g du composé obtenu à la Préparation 1.2 dans 500 ml de THF, ajoute, par petites portions et en maintenant la température entre -5°C et 0°C, 4,6 g d'hydrure d'aluminium et de lithium et laisse 1 heure sous agitation à TA. On refroidit le mélange réactionnel à 0°C, hydrolyse par ajout de 20 ml de NaOH 1N, filtre les minéraux, lave au THF et concentre sous vide le filtrat. On extrait le résidu à l'éther, lave la phase organique par une solution saturée de NaCl, sèche sur Na₂SO₄ et évapore en partie le solvant sous vide. On essore le précipité formé, le lave à l'éther et le sèche. On obtient 25 g du produit attendu.

### Préparation 2.3

### [5-(4-Bromophényl)-1-(2,4-dichlorophényl)-4-éthyl-1H-pyrazol-3-yl]méthanol.

(VI) : R₃ = -CH₂CH₃ ; R₄ = H ; R₅ = 4-Br ; R₆ = H ; R₇ = H ; R₈ = 4-Cl ; R₉ = 2-Cl.

On refroidit à -10°C un mélange de 24,6 g du composé obtenu à la Préparation 1.3 dans 200 ml de THF, ajoute 7,8 ml de triéthylamine puis, goutte à goutte, 5,38 ml de chloroformiate d'éthyle et laisse 15 minutes sous agitation à -10°C. On ajoute ensuite, en une seule fois et à une température inférieure à -10°C, 6,3 g de borohydrure de sodium puis, goutte à goutte, 100 ml de MeOH. On hydrolyse à 0°C le mélange réactionnel par ajout de 100 ml d'HCl à 10 % puis concentre sous vide. On extrait le résidu à l'AcOEt, lave deux fois la phase organique par 50 ml d'une solution saturée de NaCl, sèche sur Na₂SO₄ et évapore le solvant sous vide. On chromatographie le résidu sur gel de silice en éluant par le mélange cyclohexane/AcOEt (60/40 ; v/v). On obtient 20 g du produit attendu.

En suivant les modes opératoires décrits dans les Préparations 2 ci-dessus, on prépare les composés de formule (VI) rassemblés dans le TABLEAU II ci-après.

**TABLEAU II**

| | | | | |
|---|---|---|---|---|
| 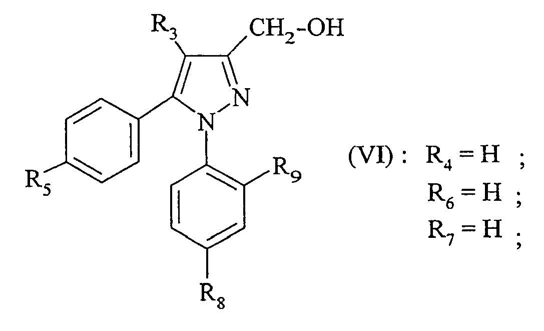 | | | | |

| Préparations | R₃ | R₅ | R₈ | R₉ |
|---|---|---|---|---|
| 2.1 | -CH₃ | Br | Cl | Cl |
| 2.2 | -CH₃ | Cl | Cl | Cl |
| 2.3 | -CH₂CH₃ | Br | Cl | Cl |
| 2.4 | -CH₃ | Cl | H | Cl |
| 2.5 | -CH₃ | F | H | Cl |
| 2.6 | -CH₃ | -OCH₃ | H | Cl |
| 2.7 | -CH₃ | -OCH₃ | H | F |
| 2.8 | -CH₃ | -OCH₃ | Cl | CI |
| 2.9 | -CH₃ | -OCH₃ | F | F |

### 3. Préparations des composés de formule (IV)

### Préparation 3.1

### 5-(4-Bromophényl)-3-(chlorométhyl)-1-(2,4-dichlorophényl)-4-méthyl-1H-pyrazole.

(IV) : R₃ = -CH₃ ; R₄ = H ; R₅ = 4-Br ; R₆ = H ; R₇ = H ; R₈ = 4-Cl ; R₉ = 2-Cl; Y = Cl.

On refroidit à 0°C, sous atmosphère d'azote, une solution de 20 g du composé obtenu à la Préparation 2.1 dans 250 ml de DCM, ajoute, par petites fractions et à une température inférieure à 5°C, 10,6 g de pentachlorure de phosphore et laisse 2 heures sous agitation en laissant remonter la température à TA. On verse le mélange réactionnel dans 150 ml d'un mélange eau/glace et laisse 10 minutes sous agitation. On extrait au DCM, lave la phase organique par une solution de NaHCO₃ à 5%, par une solution saturée de NaCl, sèche sur MgSO₄ et évapore le solvant sous vide. On obtient 24 g du produit attendu.

### Préparation 3.2

### 3-(Chlorométhyl)-5-(4-chlorophényl)-1-(2,4-dichlorophényl)-4-méthyl-1H-pyrazole.

(IV) : R₃ = -CH₃ ; R₄ = H ; R₅ = 4-Cl ; R₆ = H ; R₇ = H ; R₈ = 4-Cl ; R₉ = 2-Cl; Y = Cl.

On refroidit à 0°C, sous atmosphère d'azote, une solution de 25 g du composé obtenu à la Préparation 2.2 dans 250 ml de DCM, ajoute, par petites fractions et à une température comprise entre 0°C et 5°C, 14,8 g de pentachlorure de phosphore et laisse 3 heures sous agitation en laissant remonter la température à TA. On verse le mélange réactionnel dans 200 ml d'eau et laisse 10 minutes sous agitation. Après décantation, on lave la phase organique par une solution saturée de NaHCO₃, par une solution saturée de NaCl, sèche sur Na₂SO₄ et évapore le solvant sous vide. On obtient 25 g du produit attendu sous forme de mousses.

### Préparation 3.3

### 5-(4-Bromophényl)-3-(chlorométhyl)-1-(2,4-dichlorophényl)-4-éthyl-1H-pyrazole.

(IV) : R₃ = -CH₂CH₃ ; R₄ = H ; R₅ = 4-Br ; R₆ = H ; R₇ = H ; R₈ = 4-Cl ; R₉ = 2-Cl ; Y = Cl.

On prépare ce composé selon le mode opératoire décrit à la Préparation 3.2 à partir de 9 g du composé obtenu à la Préparation 2.3 dans 200 ml de DCM et 4,6 g de pentachlorure de phosphore. On obtient 9,4 g du produit attendu.

En suivant les modes opératoires décrits dans les Préparations 3 ci-dessus, on prépare les composés de formule (IV) rassemblés dans le TABLEAU III ci-après.

**TABLEAU III**

| | | | | | |
|---|---|---|---|---|---|
| 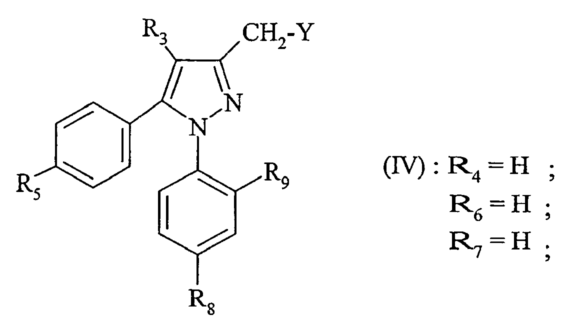 | | | | | |

| Préparations | R₃ | R₅ | R₈ | R₉ | Y |
|---|---|---|---|---|---|
| 3.1 | -CH₃ | Br | Cl | Cl | Cl |
| 3.2 | -CH₃ | Cl | Cl | Cl | Cl |
| 3.3 | -CH₂CH₃ | Br | Cl | Cl | CI |
| 3.4 | -CH₃ | Cl | H | Cl | Cl |
| 3.5 | -CH₃ | F | H | Cl | Cl |
| 3.6 | -CH₃ | -OCH₃ | H | Cl | Cl |
| 3.7 | -CH₃ | -OCH₃ | H | F | Cl |
| 3.8 | -CH₃ | -OCH₃ | Cl | Cl | Cl |
| 3.9 | -CH₃ | -OCH₃ | F | F | Cl |

### 4. Préparations des composés de formule (II).

### Préparation 4.1

### Chlorhydrate de [[5-(4-bromophényl)-1-(2,4-dichlorophényl)-4-méthyl-1H-pyrazol-3-yl]méthyl]amine.

(II), HCl : R₂ = H ; R₃ = -CH₃ ; R₄ = H ; R₅ = 4-Br ; R₆ = H ; R₇ = H ; R₈ = 4-Cl ; R₉ = 2-Cl.

A une solution de 20 g du composé obtenu à la Préparation 3.1 dans 200 ml de chloroforme, on ajoute 7 g d'hexaméthylènetétramine et laisse 5 jours sous agitation à TA. On ajoute ensuite 50 ml d'éther, essore le précipité formé et le sèche. On reprend le précipité dans 50 ml d'EtOH, ajoute 15 ml d'HCl concentré et chauffe à 50°C pendant 5 heures. On filtre l'insoluble blanc et concentre sous vide le filtrat. On reprend le résidu à l'éther, lave la phase organique par 50 ml de NaOH 5N, sèche sur Na₂SO₄ et évapore le solvant sous vide. On reprend le résidu par une solution d'éther chlorhydrique 2N, essore le précipité formé, le lave à l'éther et le sèche. On obtient 14,37 g du produit attendu.

### Préparation 4.2

### Chlorhydrate de [[5-(4-chlorophenyl)-1-(2,4-dichlorophényl)-4-méthyl-1H-pyrazol-3-yl]méthyl]amine.

(II), HCl : R₂ = H ; R₃ = -CH₃ ; R₄ = H ; R₅ = 4-Cl ; R₆ = H ; R₇ = H ; R₈ = 4-Cl ; R₉ = 2-Cl.

On prépare ce composé selon le mode opératoire décrit à la Préparation 4.1 à partir de 25 g du composé obtenu à la Préparation 3.2 dans 150 ml de chloroforme, 9 g d'hexaméthylènetétramine, 100 ml d'éther, 250 ml d'EtOH et 30 ml d'HCl concentré. On obtient 24 g du produit attendu.

### Préparation 4.3

### Chlorhydrate de [[5-(4-bromophényl)-1-(2,4-dichlorophényl)-4-éthyl-1H-pyrazol-3-yl]méthyl]amine.

(II), HCl : R₂ = H ; R₃ = -CH₂CH₃ ; R₄ = H ; R₅ = 4-Br ; R₆ = H ; R₇ = H ; R₈ = 4-Cl ; R₉ = 2-Cl.

A une solution de 9 g du composé obtenu à la Préparation 3.3 dans 100 ml de chloroforme on ajoute 2,9 g d'hexaméthylènetétramine et laisse 10 jours sous agitation à TA. On ajoute ensuite 50 ml d'éther, laisse 10 minutes sous agitation, concentre de moitié sous vide le mélange réactionnel, rajoute 50 ml d'éther et essore le précipité formé. On reprend le précipité dans 50 ml d'EtOH, ajoute 15 ml d'HCl concentré et chauffe à reflux pendant 2 heures. On filtre l'insoluble blanc et concentre sous vide le filtrat. On extrait le résidu à l'éther, lave la phase organique par 20 ml de NaOH 1N, par une solution saturée de NaCl, sèche sur Na₂SO₄ et évapore le solvant sous vide. On reprend le résidu par une solution d'éther chlorhydrique 2N et essore le précipité formé. On obtient 8,5 g du produit attendu.

En suivant les modes opératoires décrits dans les Préparations 4 ci-dessus, on prépare les composés de formule (II) rassemblés dans le TABLEAU IV ci-après.

**TABLEAU IV**

| | | | | | |
|---|---|---|---|---|---|
| 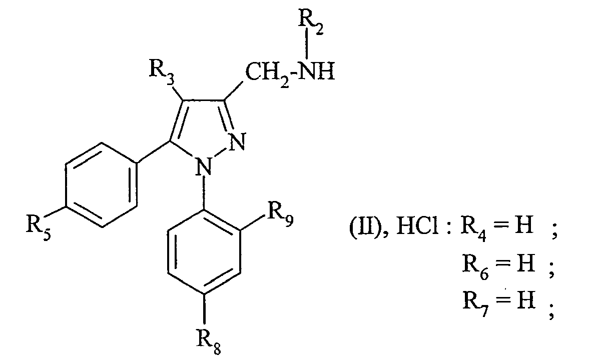 | | | | | |

| Préparations | R₂ | R₃ | R₅ | R₈ | R₉ |
|---|---|---|---|---|---|
| 4.1 | H | -CH₃ | Br | Cl | Cl |
| 4.2 | H | -CH₃ | Cl | Cl | Cl |
| 4.3 | H | -CH₂CH₃ | Br | Cl | Cl |
| 4.4 | H | -CH₃ | Cl | H | Cl |
| 4.5 | H | -CH₃ | F | H | Cl |
| 4.6 | H | -CH₃ | -OCH₃ | H | Cl |
| 4.7 | H | -CH₃ | -OCH₃ | H | F |
| 4.8 | H | -CH₃ | -OCH₃ | Cl | Cl |
| 4.9 | H | -CH₃ | -OCH₃ | F | F |

### 5. Préparations des composés de formule (III).

### Chlorure de cyclohexanesulfonyle.

(III) : 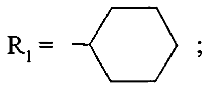 Hal = Cl.

On refroidit à 0°C un mélange de 25 g de cyclohexanethiol dans 83 ml d'acide acétique et 4 ml d'eau, puis fait barboter pendant 3 heures du chlore gazeux jusqu'à ce que la couleur jaune persiste. On verse le mélange réactionnel dans l'eau, extrait trois fois à l'éther, lave deux fois les phases organiques jointes à l'eau, sèche sur Na₂SO₄ et évapore le solvant sous vide. On distille le résidu sous une pression de 4 Pa et obtient 13,3 g du produit attendu, Eb = 154°C.

### EXEMPLE 1 : Composé N° 8

### N-[[5-(4-Bromophényl)-1-(2,4-dichlorophényl)-4-méthyl-1H-pyrazol-3-yl] méthyl]-4-(trifluorométhyl)benzènesulfonamide.

(I) :

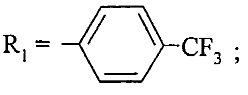

R₂ = H ; R₃ = -CH₃ ; R₄ = H ; R₅ = 4-Br ; R₆ = H ; R₇ = H ; R₈ = 4-Cl ; R₉ = 2-Cl.

A une solution de 0,7 g du composé obtenu à la Préparation 4.1 dans 20 ml de DCM on ajoute 0,42 ml de triéthylamine puis, goutte à goutte, 0,39 g de chlorure de 4-(trifluorométhyl)benzènesulfonyle et laisse une nuit sous agitation à TA. On ajoute 10 ml d'eau et laisse 10 minutes sous agitation. Après décantation, on lave la phase organique deux fois par une solution saturée de NaHCO₃, deux fois par une solution tampon pH = 2, deux fois par une solution saturée de NaCl, sèche sur Na₂SO₄ et évapore le solvant sous vide. On cristallise le produit obtenu dans un mélange cyclohexane/AcOEt (95/5 ; v/v). On obtient 0,75 g du produit attendu, F = 195°C.

RMN ¹H : DMSO-d₆ : δ (ppm) : 1,95 : s : 3H ; 4,15 : s : 2H ; 7,0 : d : 2H ; 7,2-8,1 : m : 9H ; 8,5 : s : 1H.

### EXEMPLE 2 : Composé N° 28

### N-[[5-(4-Bromophényl)-1-(2,4-dichlorophényl)-4-éthyl-1H-pyrazol-3-yl] méthyl]-3-chlorobenzènesulfonamide.

(I) :

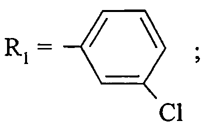

R₂ = H ; R₃ = -CH₂CH₃ ; R₄ = H ; R₅ = 4-Br ;
R₆ = H ; R₇ = H ; R₈ = 4-Cl ; R₉ = 2-Cl.

A une solution de 0,7 g du composé obtenu à la Préparation 4.3 dans 30 ml de DCM, on ajoute 0,4 ml de triéthylamine puis, goutte à goutte, 0,32 g de chlorure de 3-chlorobenzènesulfonyle et laisse une nuit sous agitation à TA. On concentre le mélange réactionnel sous vide, extrait le résidu à l'AcOEt, lave la phase organique deux fois par une solution saturée de NaHCO₃, deux fois par une solution tampon pH = 2, deux fois par une solution saturée de NaCl, sèche sur Na₂SO₄ et évapore le solvant sous vide. On chromatographie le résidu sur gel de silice en éluant par le mélange cyclohexane/AcOEt de (90/10 ; v/v) à (75/25 ; v/v). On reprend le produit obtenu dans 1,5 ml d'AcOEt, ajoute du cyclohexane, essore le précipité formé et le sèche. On obtient 0,31 g du produit attendu, F = 169°C.

RMN ¹H : DMSO-d₆ : δ (ppm) : 1,0 : t : 3H ; 2,4 : q : 2H ; 4,15 : s : 2H ; 7,0 : d : 2H ; 7,2-7,9 : m : 9H ; 8,4 : s : 1H.

### EXEMPLE 3 : Composé N° 42

### N-[[5-(4-Bromophényl)-1-(2,4-dichlorophényl)-4-éthyl-1H-pyrazol-3-yl] méthyl]-1-[3-(trifluorométhyl)phényl]méthanesulfonamide.

(I) :

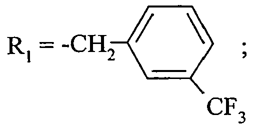

R₂ = H ; R₃ = -CH₂CH₃ ; R₄ = H ; R₅ = 4-Br ;
R₆ = H ; R₇ = H ; R₈ = 4-Cl ; R₉ = 2-Cl.

A une solution de 0,5 g du composé obtenu à la Préparation 4.3 dans 25 ml de DCM, on ajoute 0,3 ml de triéthylamine puis 0,28 g de chlorure de [3-(trifluorométhyl)phényl]méthanesulfonyle et laisse une nuit sous agitation à TA. On concentre le mélange réactionnel sous vide, reprend le résidu à l'eau, extrait à l'AcOEt, lave la phase organique par une solution saturée de NaHCO₃, par une solution tampon pH = 2, par une solution saturée de NaCl, sèche sur Na₂SO₄ et évapore le solvant sous vide. On chromatographie le résidu sur gel de silice en éluant par le mélange cyclohexane/AcOEt de (90/10 ; v/v) à (85/15 ; v/v). On obtient 0,3 g du composé attendu, F = 108°C.

RMN ¹H : DMSO-d₆ : δ (ppm) : 1,03 : t : 3H ; 2,5 : mt : 2H ; 4,23 : d : 2H ; 4,5 : s: 2H ; 7,13 : d : 2H ; 7,4-7,75 : m : 9H ; 7,80 : t : 1H.

### EXEMPLE 4 : Composé N° 73

### N-[[5-(4-Bromophényl)-1-(2,4-dichlorophényl)-4-éthyl-1H-pyrazol-3-yl] méthyl]-3-chloro-N-méthylbenzènesulfonamide.

(I) : 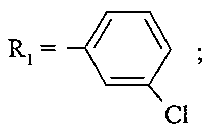 R₂ = CH₃ ; R₃ = -CH₂CH₃ ; R₄ = H ; R₅ = 4-Br ;
R₆ = H ; R₇ = H ; R₈ = 4-Cl ; R₉ = 2-Cl.

A un mélange de 0,41 g du composé N° 28 et 0,1 g de K₂CO₃ dans 34 ml de DMF on ajoute 0,05 ml d'iodure de méthyle puis chauffe à reflux pendant 2 heures et laisse une nuit sous agitation à TA. On concentre le mélange réactionnel sous vide, reprend le résidu à l'eau, extrait au DCM, lave la phase organique par une solution tampon pH = 2, par une solution saturée de NaHCO₃, par une solution saturée de NaCl, sèche sur Na₂SO₄ et évapore le solvant sous vide. On chromatographie le résidu sur gel de silice en éluant par le mélange cyclohexane/AcOEt de (90/10 ; v/v) à (80/20 ; v/v). On obtient 0,263 g du composé attendu après séchage sous vide, F = 78°C.

Le tableau qui suit illustre les structures chimiques et les propriétés physiques de quelques exemples de composés selon l'invention. Dans ce tableau :
- dans la colonne "sel", "-" représente un composé sous forme de base libre, alors que "HCl" représente un composé sous forme de chlorhydrate ;
- dans la colonne, "méthode" représente une des méthodes d'analyse utilisée pour déterminer le pic moléculaire MH⁺ et le temps de rétention telle que décrit précédemment.

**TABLEAU 1**

| | | | | | | | |
|---|---|---|---|---|---|---|---|
| 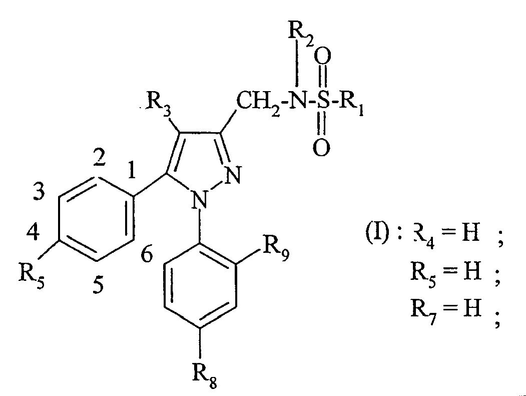 | | | | | | | |

| Composés N° | R₁ | R₂ | R₃ | R₅ | R₈ | R₉ | Sel; F°C ; MH⁺ ; tr (méthode) |
|---|---|---|---|---|---|---|---|
| 1 | -CH₂CH₂CH₂CH₃ | H | -CH₃ | Br | Cl | Cl | - |
| (a) | | | | | | | - |
| | | | | | | | 530,36 ; 2,33 |
| | | | | | | | (méthode A) |
| 2 | 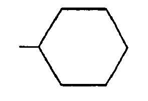 | H | -CH₃ | Br | Cl | Cl | - |
| (a) | | | | | | | 98 ; |
| | | | | | | | |
| 3 | 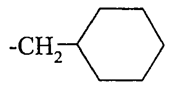 | H | -CH₃ | Br | Cl | Cl | - |
| (a) | | | | | | | 145 ; |
| | | | | | | | |
| 4 | 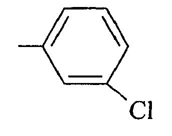 | H | -CH₃ | Br | Cl | Cl | - |
| | | | | | | | - |
| | | | | | | | 584,32 ; 2,41 |
| | | | | | | | (méthode A) |
| 5 | 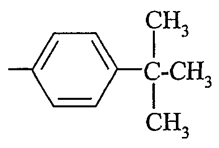 | H | -CH₃ | Br | Cl | Cl | - |
| (a) | | | | | | | - |
| | | | | | | | 606,05 ; 2,31 |
| | | | | | | | (méthode A) |
| 6 | 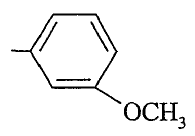 | H | -CH₃ | Br | Cl | Cl | - |
| (a) | | | | | | | - |
| | | | | | | | 579,97; 2,21 |
| | | | | | | | (méthode A) |
| 7 | 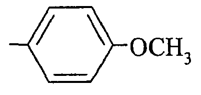 | H | -CH₃ | Br | Cl | Cl | - |
| (a) | | | | | | | 95 ; |
| | | | | | | | - |
| 8 | 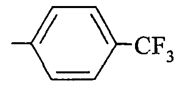 | H | -CH₃ | Br | Cl | Cl | - |
| | | | | | | | 195 ; |
| | | | | | | | - |
| | | | | | | | - |
| 9 | 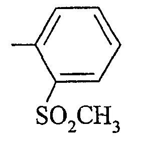 | H | -CH₃ | Br | Cl | Cl | - |
| (a) | | | | | | | - |
| | | | | | | | 672,93 ; 2,21 |
| | | | | | | | (méthode A) |
| 10 | 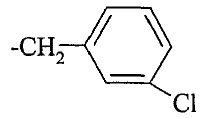 | H | -CH₃ | Br | Cl | Cl | - |
| (a) | | | | | | | 95 ; |
| | | | | | | | - |
| 11 | 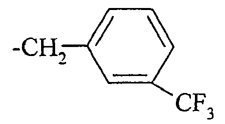 | H | -CH₃ | Br | Cl | Cl | - |
| (a) | | | | | | | 96 ; |
| | | | | | | | - |
| 12 | 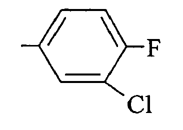 | H | -CH₃ | Br | Cl | Cl | - |
| (a) | | | | | | | - |
| | | | | | | | 602,29 ; 2,43 |
| | | | | | | | (méthode A) |
| 13 | -CH₂CH₂CH₂CH₃ | H | -CH₃ | Cl | Cl | Cl | - |
| (b) | | | | | | | - |
| | | | | | | | 486,42 ; 2,30 |
| | | | | | | | (méthode A) |
| 14 | 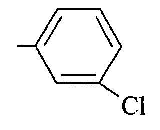 | H | -CH₃ | Cl | Cl | Cl | - |
| (b) | | | | | | | - |
| | | | | | | | 540,36 ; 2,39 |
| | | | | | | | (méthode A) |
| 15 | 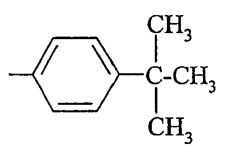 | H | -CH₃ | Cl | Cl | Cl | - |
| (b) | | | | | | | 115 ; |
| | | | | | | | - |
| 16 | 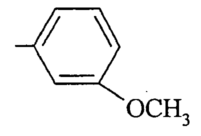 | H | -CH₃ | Cl | Cl | CI | - |
| (b) | | | | | | | - |
| | | | | | | | 536,06 ; 2,20 |
| | | | | | | | (méthode A) |
| 17 | 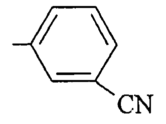 | H | -CH₃ | Cl | Cl | CI | - |
| (b) | | | | | | | - |
| | | | | | | | 531,02 ; 2,16 |
| | | | | | | | (méthode A) |
| 18 | 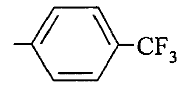 | H | -CH₃ | Cl | Cl | Cl | - |
| (b) | | | | | | | 189 ; |
| | | | | | | | - |
| 19 | 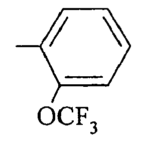 | H | -CH₃ | Cl | Cl | Cl | - |
| (b) | | | | | | | - |
| | | | | | | | 590,01 ; 2,25 |
| | | | | | | | (méthode A) |
| 20 | 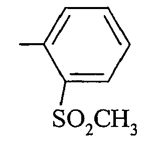 | H | -CH₃ | Cl | Cl | Cl | Cl |
| (b) | | | | | | | |
| | | | | | | | |
| | | | | | | | |
| 21 | 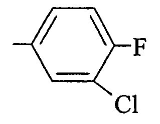 | H | -CH₃ | Cl | Cl | Cl | - |
| (b) | | | | | | | - |
| | | | | | | | 558,4 ; 2,41 |
| | | | | | | | (méthode A) |
| 22 | 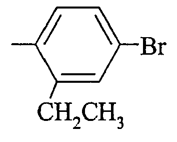 | H | -CH₃ | Cl | Cl | Cl | - |
| (b) | | | | | | | - |
| | | | | | | | 611,96 ; 2,33 |
| | | | | | | | (méthode A) |
| 23 | -CH₂CH₃ | H | -CH₂CH₃ | Br | Cl | Cl | - |
| (c) | | | | | | | 125 ; |
| | | | | | | | - |
| 24 | 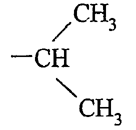 | H | -CH₂CH₃ | Br | Cl | Cl | - |
| (c) | | | | | | | 157 ; |
| | | | | | | | - |
| 25 | -CH₂CH₂CH₂CH₃ | H | -CH₂CH₃ | Br | Cl | Cl | - |
| (c) | | | | | | | 65 ; |
| | | | | | | | - |
| 26 | 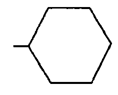 | H | -CH₂CH₃ | Br | Cl | Cl | - |
| (c) | | | | | | | 98 ; |
| | | | | | | | - |
| 27 | 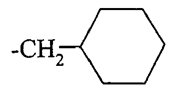 | H | -CH₂CH₃ | Br | Cl | Cl | - |
| (c) | | | | | | | 72 ; |
| | | | | | | | - |
| 28 | 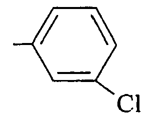 | H | -CH₂CH₃ | Br | Cl | Cl | - |
| (c) | | | | | | | 169 ; |
| | | | | | | | - |
| 29 | 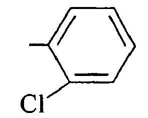 | H | -CH₂CH₃ | Br | Cl | Cl | - |
| (c) | | | | | | | 135 ; |
| | | | | | | | - |
| 30 | 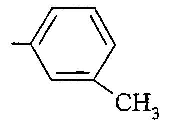 | H | -CH₂CH₃ | Br | Cl | Cl | - |
| (c) | | | | | | | 165 ; |
| | | | | | | | - |
| 31 | 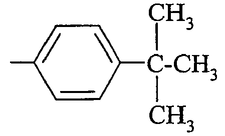 | H | -CH₂CH₃ | Br | Cl | Cl | - |
| (c) | | | | | | | 82 ; |
| | | | | | | | - |
| 32 | 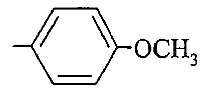 | H | -CH₂CH₃ | Br | Cl | Cl | - |
| (c) | | | | | | | 121 ; |
| | | | | | | | - |
| 33 | 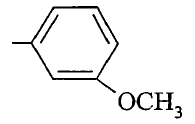 | H | -CH₂CH₃ | Br | Cl | Cl | - |
| (c) | | | | | | | 131 ; |
| | | | | | | | - |
| 34 | 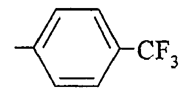 | H | -CH₂CH₃ | Br | Cl | Cl | - |
| (c) | | | | | | | 65 ; |
| | | | | | | | - |
| 35 | 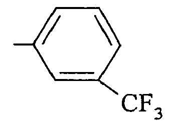 | H | -CH₂CH₃ | Br | Cl | Cl | - |
| (c) | | | | | | | 95 ; |
| | | | | | | | - |
| 36 | 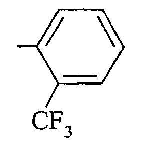 | H | -CH₂CH₃ | Br | Cl | Cl | - |
| (c) | | | | | | | 168 ; |
| | | | | | | | - |
| 37 | 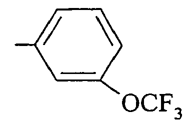 | H | -CH₂CH₃ | Br | Cl | Cl | - |
| (c) | | | | | | | 75 ; |
| | | | | | | | - |
| 38 | 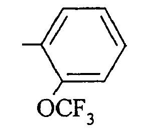 | H | -CH₂CH₃ | Br | Cl | Cl | - |
| (c) | | | | | | | 145 ; |
| | | | | | | | - |
| 39 | 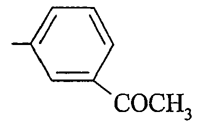 | H | -CH₂CH₃ | Br | CI | Cl | - |
| (c) | | | | | | | 88; |
| | | | | | | | - |
| 40 | 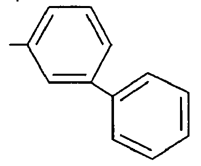 | H | -CH₂CH₃ | Br | Cl | Cl | - |
| (c) | | | | | | | 183 ; |
| | | | | | | | - |
| 41 | 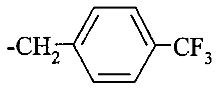 | H | -CH₂CH₃ | Br | Cl | Cl | - |
| (c) | | | | | | | 87 ; |
| | | | | | | | - |
| 42 | 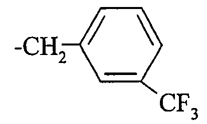 | H | -CH₂CH₃ | Br | Cl | Cl | - |
| (c) | | | | | | | 108 ; |
| | | | | | | | - |
| 43 | 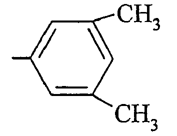 | H | -CH₂CH₃ | Br | Cl | Cl | - |
| (c) | | | | | | | 153 ; |
| | | | | | | | - |
| 44 | 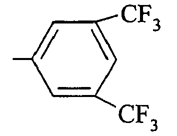 | H | -CH₂CH₃ | Br | Cl | Cl | - |
| (c) | | | | | | | 105 ; |
| | | | | | | | |
| 45 | 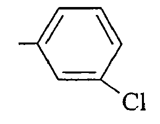 | H | -CH₃ | Cl | H | Cl | - |
| (d) | | | | | | | - |
| | | | | | | | 506 ; 10,9 |
| | | | | | | | (méthode B) |
| 46 | 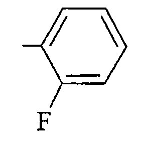 | H | -CH₃ | Cl | H | Cl | - |
| (d) | | | | | | | - |
| | | | | | | | 490 ; 10,4 |
| | | | | | | | (méthode B) |
| 47 | 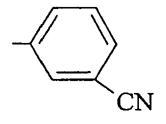 | H | -CH₃ | Cl | H | Cl | - |
| (d) | | | | | | | - |
| | | | | | | | 497 ; 10,2 |
| | | | | | | | (méthode B) |
| 48 | 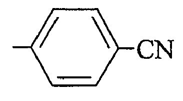 | H | -CH₃ | Cl | H | Cl | - |
| (d) | | | | | | | 172 ; |
| | | | | | | | 497 ; 10,19 |
| | | | | | | | (méthode B) |
| 49 | 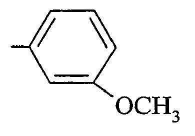 | H | -CH₃ | Cl | H | Cl | - |
| (d) | | | | | | | 151 ; |
| | | | | | | | 502 ; 10,5 |
| | | | | | | | (méthode B) |
| 50 | 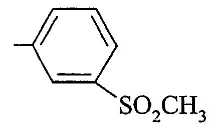 | H | -CH₃ | Cl | H | Cl | - |
| (d) | | | | | | | 110 ; |
| | | | | | | | 550 ; 9,77 |
| | | | | | | | (méthode B) |
| 51 | 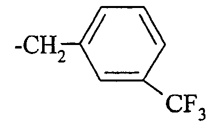 | H | -CH₃ | Cl | H | Cl | - |
| (d) | | | | | | | - |
| | | | | | | | 554; 11,1 |
| | | | | | | | (méthode B) |
| 52 | 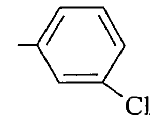 | H | -CH₃ | F | H | Cl | - |
| (e) | | | | | | | 70 ; |
| | | | | | | | 490 ; 10,4 |
| | | | | | | | (méthode B) |
| 53 | 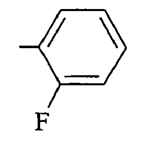 | H | -CH₃ | F | H | Cl | - |
| (e) | | | | | | | 147 ; |
| | | | | | | | 474 ; 9,9 |
| | | | | | | | (méthode B) |
| 54 | 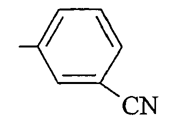 | H | -CH₃ | F | H | Cl | - |
| (e) | | | | | | | 80 ; |
| | | | | | | | 481 ; 9,7 |
| | | | | | | | (méthode B) |
| 55 | 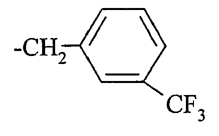 | H | -CH₃ | F | H | Cl | - |
| (e) | | | | | | | 66 ; |
| | | | | | | | 538 ; 10,6 |
| | | | | | | | (méthode B) |
| 56 | 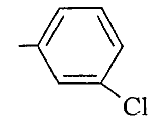 | H | -CH₃ | -OCH₃ | H | Cl | - |
| (f) | | | | | | | 73 ; |
| | | | | | | | 502 ; 10,1 |
| | | | | | | | (méthode B) |
| 57 | 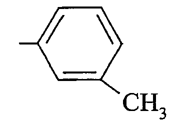 | H | -CH₃ | -OCH₃ | H | Cl | - |
| (f) | | | | | | | 66 ; |
| | | | | | | | 482 ; 10 |
| | | | | | | | (méthode B) |
| 58 | 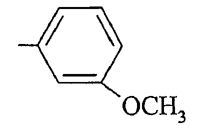 | H | -CH₃ | -OCH₃ | H | Cl | - |
| (f) | | | | | | | 65 ; |
| | | | | | | | 498 ; 9,7 |
| | | | | | | | (méthode B) |
| 59 | 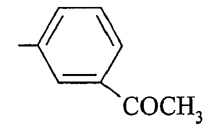 | H | -CH₃ | -OCH₃ | H | Cl | - |
| (f) | | | | | | | 89 ; |
| | | | | | | | 510 ; 9,3 |
| | | | | | | | (méthode B) |
| 60 | 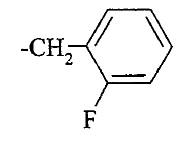 | H | -CH₃ | -OCH₃ | H | Cl | - |
| (f) | | | | | | | 64 ; |
| | | | | | | | 500 ; 9,8 |
| | | | | | | | (méthode B) |
| 61 | 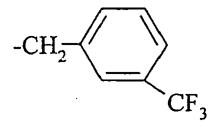 | H | -CH₃ | -OCH₃ | H | CI | - |
| (f) | | | | | | | 61; |
| | | | | | | | 550; 10,3 |
| | | | | | | | (méthode B) |
| 62 | 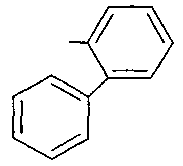 | H | -CH₃ | -OCH₃ | H | Cl | - |
| (f) | | | | | | | 140 ; |
| | | | | | | | 544 ; 10,6 |
| | | | | | | | (méthode D) |
| 63 | 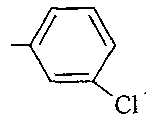 | H | -CH₃ | -OCH₃ | H | F | - |
| (g) | | | | | | | - |
| | | | | | | | 486 ; 9,9 |
| | | | | | | | (méthode B) |
| 64 | 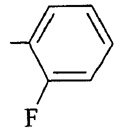 | H | -CH₃ | -OCH₃ | H | F | - |
| (g) | | | | | | | 225 ; |
| | | | | | | | 470 ; 9,4 |
| | | | | | | | (méthode B) |
| 65 | 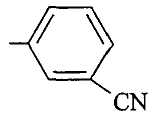 | H | -CH₃ | -OCH₃ | H | F | - |
| (g) | | | | | | | - |
| | | | | | | | 114 ; |
| | | | | | | | 477 ; 9,3 |
| | | | | | | | (méthode B) |
| 66 | 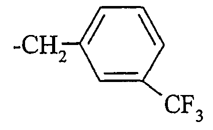 | H | -CH₃ | -OCH₃ | H | F | - |
| (g) | | | | | | | 60 ; |
| | | | | | | | 534; 10,3 |
| | | | | | | | (méthode B) |
| 67 | 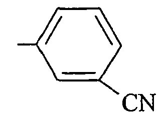 | H | -CH₃ | Br | Cl | Cl | - |
| (a) | | | | | | | 108 ; |
| | | | | | | | 573 ; 11 |
| | | | | | | | (méthode B) |
| 68 | 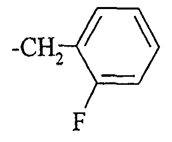 | H | -CH₂CH₃ | Br | Cl | Cl | - |
| (c) | | | | | | | 69 ; |
| | | | | | | | 596 ; 11,6 |
| | | | | | | | (méthode B) |
| 69 | 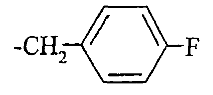 | H | -CH₂CH₃ | Br | Cl | Cl | - |
| (c) | | | | | | | 76 ; |
| | | | | | | | 596; 11,3 |
| | | | | | | | (méthode B) |
| 70 | 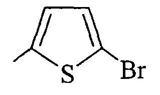 | H | -CH₂CH₃ | Br | CI | Cl | - |
| (c) | | | | | | | 143 ; |
| | | | | | | | 648 ; 18,9 |
| | | | | | | | (méthode E) |
| 71 | 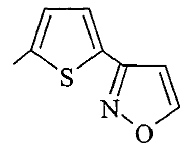 | H | -CH₂CH₃ | Br | Cl | Cl | - |
| (c) | | | | | | | 143 ; |
| | | | | | | | 648 ; 18,9 |
| | | | | | | | (méthode B) |
| 72 | 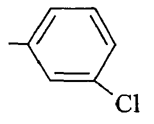 | -CH₃ | -CH₂CH₃ | Br | Cl | Cl | - |
| | | | | | | | 78 ; |
| | | | | | | | 612 ; 12,8 |
| | | | | | | | (méthode B) |
| 73 | 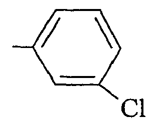 | H | -CH₃ | -OCH₃ | Cl | Cl | - |
| (h) | | | | | | | 173 ; |
| | | | | | | | 536 ; 10,8 |
| | | | | | | | (méthode B) |
| 74 | 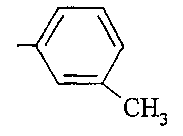 | H | -CH₃ | -OCH₃ | Cl | Cl | - |
| (h) | | | | | | | 155 ; |
| | | | | | | | 516 ; 10,6 |
| | | | | | | | (méthode B) |
| 75 | 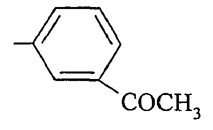 | H | -CH₃ | -OCH₃ | Cl | Cl | - |
| (h) | | | | | | | 118 ; |
| | | | | | | | 544 ; 10 |
| | | | | | | | (méthode B) |
| 76 | 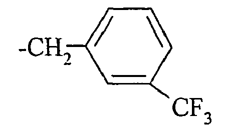 | H | -CH₃ | -OCH₃ | Cl | Cl | - |
| (h) | | | | | | | 78 ; |
| | | | | | | | 584 ; 11 |
| | | | | | | | (méthode B) |
| 77 | 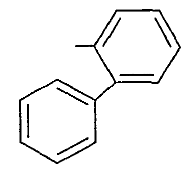 | H | -CH₃ | -OCH₃ | Cl | Cl | - |
| (h) | | | | | | | 80 ; |
| | | | | | | | 578 ; 11,5 |
| | | | | | | | (méthode B) |
| 78 | 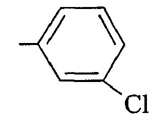 | H | -CH₃ | -OCH₃ | F | F | - |
| (i) | | | | | | | - |
| | | | | | | | 504; 10,1 |
| | | | | | | | (méthode B) |
| 79 | 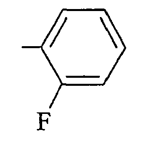 | H | -CH₃ | -OCH₃ | F | F | - |
| (i) | | | | | | | - |
| | | | | | | | 488 ; 9,6 |
| | | | | | | | (méthode B) |
| 80 | 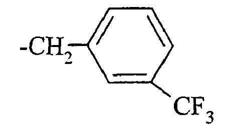 | H | -CH₃ | -OCH₃ | F | F | - |
| (i) | | | | | | | 69 |
| | | | | | | | 552 ; 10,41 |
| | | | | | | | (méthode B) |

| | | | | | | | |
|---|---|---|---|---|---|---|---|
| (a) Composé préparé selon le mode opératoire décrit à l'Exemple 1 à partir du composé obtenu à la Préparation 4.1 et du composé de formule (III) correspondant. (b) Composé préparé selon le mode opératoire décrit à l'Exemple 1 à partir du composé obtenu à la Préparation 4.2 et du composé de formule (III) correspondant. (c) Composé préparé selon le mode opératoire décrit à l'Exemple 1 à partir du composé obtenu à la Préparation 4.3 et du composé de formule (III) correspondant. (d) Composé préparé selon le mode opératoire décrit à l'Exemple 1 à partir du composé obtenu à la Préparation 4.4 et du composé de formule (III) correspondant. (e) Composé préparé selon le mode opératoire décrit à l'Exemple 1 à partir du composé obtenu à la Préparation 4.5 et du composé de formule (III) correspondant. (f) Composé préparé selon le mode opératoire décrit à l'Exemple 1 à partir du composé obtenu à la Préparation 4.6 et du composé de formule (III) correspondant. (g) Composé préparé selon le mode opératoire décrit à l'Exemple 1 à partir du composé obtenu à la Préparation 4.7 et du composé de formule (III) correspondant. (h) Composé préparé selon le mode opératoire décrit à l'Exemple 1 à partir du composé obtenu à la Préparation 4.8 et du composé de formule (III) correspondant. (i) Composé préparé selon le mode opératoire décrit à l'Exemple 1 à partir du composé obtenu à la Préparation 4.9 et du composé de formule (III) correspondant. | | | | | | | |

Les composés de formule (I) possèdent une très bonne affinité *in vitro* (IC₅₀ ≤ 5.10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

La toxicité des composés de formule (I) est compatible avec leur utilisation en tant que médicament.

Ainsi, selon un autre de ses aspects, l'invention a pour objet des médicaments qui comprennent un composé de formule (I), ou un sel d'addition de ce dernier à un acide pharmaceutiquement acceptable, ou encore un solvat ou un hydrate du composé de formule (I).

Ainsi les composés selon l'invention peuvent être utilisés chez l'homme ou chez l'animal, dans le traitement ou la prévention de maladies impliquant les récepteurs aux cannabinoïdes CB₁.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles de l'attention et de l'hyperactivité (TDAH) chez les enfants hyperkinétiques (MBD) ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, les composés de formule (I) peuvent être utiles comme neuroprotecteurs, dans le traitement de l'ischémie, des traumatismes crâniens et le traitement des maladies neurodégénératives : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire.

Les composés de formule (I) selon l'invention peuvent être utilisés comme médicaments dans le traitement des troubles de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement de l'obésité et des risques associés à l'obésité, notamment les risques cardio-vasculaires. De plus, les composés de formule (I) selon l'invention peuvent être utilisés en tant que médicaments dans le traitement des troubles gastro-intestinaux, des troubles diarrhéiques, des ulcères, des vomissements, des troubles vésicaux et urinaires, des troubles d'origine endocrinienne, des troubles cardio-vasculaires, de l'hypotension, du choc hémorragique, du choc septique, de la cirrhose chronique du foie, de la stéatose hépatique, de la stéatohépatite, de l'asthme, du syndrome de Raynaud, du glaucome, des troubles de la fertilité, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement de l'ostéoporose.

Selon la présente invention, les composés de formule (I) sont tout particulièrement utiles pour le traitement des troubles psychotiques, en particulier la schizophrénie, les troubles de l'attention et de l'hyperactivité (TDAH) chez les enfants hyperkinétiques (MBD) ; pour le traitement des troubles de l'appétit et de l'obésité ; pour le traitement des déficits mnésiques et cognitifs ; pour le traitement de la dépendance alcoolique, de la dépendance nicotinique, c'est à dire pour le sevrage alcoolique et pour le sevrage tabagique.

Selon un de ses aspects, la présente invention est relative à l'utilisation d'un composé de formule (I), de ses sels pharmaceutiquement acceptables et de leurs solvats ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | : 50,0 mg |
| Mannitol | : 223,75 mg |
| Croscarmellose sodique | : 6,0 mg |
| Amidon de maïs | : 15,0 mg |
| Hydroxypropyl-méthylcellulose | : 2,25 mg |
| Stéarate de magnésium | : 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également l'ulilisation d'un composé de formule (I) pour la préparation d'un médicament destiné à une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 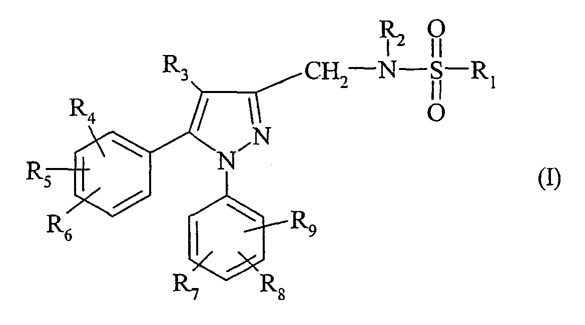 dans laquelle :
R₁ représente
. un (C₁-C₆)alkyle ;
. un (C₃-C₇)cycloalkyle non substitué ou substitué une ou plusieurs fois par un groupe (C₁-C₃)alkyle ;
. un (C₃-C₇)cycloalkylméthyle non substitué ou substitué une ou plusieurs fois sur le carbocycle par un (C₁-C₃)alkyle ;
. un phényle non substitué ou mono-, di- ou trisubstitué par un substituant choisi indépendamment parmi un atome d'halogène, un (C₁-C₄)alkyle, un (C₁-C₃)alcoxy, un cyano, un radical trifluorométhyle, un radical trifluorométhoxy, un groupe S(O)ₙAlk, un groupe (C₁-C₃)alkylcarbonyle, un phényle ;
. un benzyle non substitué ou mono- ou disubstitué par un substituant choisi indépendamment parmi un atome d'halogène, un (C₁-C₃)alkyle, un (C₁-C₃)alcoxy ; un radical trifluorométhyle ;
. un thiényle non substitué ou substitué par un atome d'halogène ou par un isoxazolyle ;
- R₂ représente un atome d'hydrogène ou un (C₁-C₃)alkyle ;
- R₃ représente un atome d'hydrogène ou un (C₁-C₅)alhyle ;
- R₄, R₅, R₆, R₇, R₈ et R₉ représentent chacun indépendamment un atome d'hydrogène, un atome d'halogène, un (C₁-C₇)alkyle, un (C₁-C₅)alcoxy, un radical trifluorométhyle ou un groupe S(O)ₙAlk ;
- n représente 0, 1 ou 2 ;
- Alk représente un (C₁-C₄)alkyle.
à l'état de base ou de sel d'addition à un acide, ainsi qu'à l'état d'hydrate ou de solvat.

2. Composé de formule (I) selon la revendication 1 dans laquelle :
- R₁ représente
. un éthyle, un isopropyle, un n-butyle ;
. un cyclohexyle ;
. un cyclohexylméthyle ;
. un 2-chlorophényle, un 3-chlorophényle, un 2-fluorophényle, un 3-chloro-4-fluorophényle, un 4-bromo-2-éthylphényle, un 3-méthylphényle, un 4-*tert-*butylphényle, un 3,5-diméthylphényle, un 3-méthoxyphényle, un 4-méthoxyphényle, un 3-cyanophényle, un 4-cyanophényle, un 2-(trifluorométhyl)phényle, un 3-(trifluorométhyl)phényle, un 4-(trifluorométhyl)phényle, un 3,5-bis(trifluorométhyl)phényle, un 2-trifluorométhoxy)phényle, un 3-(trifluorométhoxy)phényle, un 2-(méthylsulfonyl)phényle, un 3-(méthylsulfonyl)phényle, un 3-acétylphényle, un 3-biphényle, un 2-biphényle ;
. un 3-chlorobenzyle, un 2-fluorobenzyle, un 4-fluorobenzyle, un 3-(trifluorométhyl)benzyle, un 4-(trifluorométhyl)benzyle ;
. un 5-bromo-2-thiényle ; un 5-isoxazol-3-yl-2-thiényle ;
- R₂ représente un atome d'hydrogène ou un méthyle ;
- R₃ représente un méthyle ou un éthyle ;
- R₄ représente un atome d'hydrogène ;
- R₅ est en position -4- du phényle et représente un atome de brome, de chlore, de fluor ou un méthoxy ;
- R₆ représente un atome d'hydrogène ;
- R₇ représente un atome d'hydrogène ;
- R₈ est en position -4- du phényle et représente un atome d'hydrogène, un atome de chlore, un atome de fluor ;
- R₉ est en position -2- du phényle et représente un atome de chlore ou de fluor ; à l'état de base ou de sel d'addition à un acide, ainsi qu'à l'état d'hydrate ou de solvat.

3. Composé de formule (I) selon la revendication 1 choisi parmi :
- N-[[5-(4-bromophényl)-1-(2,4-dichlorophényl)-4-méthyl-1*H*-pyrazol-3-yl]méthyl]butane-1-sulfonamide ;
- N-[[5-(4-bromophényl)-1-(2,4-dichlorophényl)-4-méthyl-1*H*-pyrazol-3-yl]méthyl]cyclohexanesulfonamide ;
- N-[[5-(4-bromophényl)-1-(2,4-dichlorophényl)-4-méthyl-1*H*-pyrazol-3-yl]méthyl]cyclohexylméthanesulfonamide ;
- N-[[5-(4-bromophényl)-1-(2,4-dichlorophényl)-4-méthyl-1*H*-pyrazol-3-yl]méthyl]-3-chlorobenzènesulfonamide ;
- N-[[5-(4-bromophényl)-1-(2,4-dichlorophényl)-4-méthyl-1*H*-pyrazol-3-yl]méthyl]-4-*tert*-butylbenzènesulfonamide ;
- N-[[5-(4-bromophényl)-1-(2,4-dichlorophényl)-4-méthyl-1*H*-pyrazol-3-yl]méthyl]-3-méthoxybenzènesulfonamide ;
- N-[[5-(4-bromophényl)-1-(2,4-dichlorophényl)-4-méthyl-1*H*-pyrazol-3-yl]méthyl]-4-méthoxybenzènesulfonamide ;
- N-[[5-(4-bromophényl)-1-(2,4-dichlorophényl)-4-méthyl-1*H*-pyrazol-3-yl]méthyl]-4-(trifluorométhyl)benzènesulfonamide ;
- N-[[5-(4-bromophényl)-1-(2,4-dichlorophényl)-4-méthyl-1*H*-pyrazol-3-yl]méthyl]-2-(méthylsulfonyl)benzènesulfonamide ;
- N-[[5-(4-bromophényl)-1-(2,4-dichlorophényl)-4-méthyl-1*H*-pyrazol-3-yl]méthyl]-1-(3-chlorophényl)méthanesulfonamide ;
- N-[[5-(4-bromophényl)-1-(2,4-dichlorophényl)-4-méthyl-1*H*-pyrazol-3-yl]méthyl]-1-[3-(trifluorométhyl)phényl]méthanesulfonamide ;
- N-[[5-(4-bromophényl)-1-(2,4-dichlorophényl)-4-méthyl-1*H*-pyrazol-3-yl]méthyl]-3-chloro-4-fluorobenzènesulfonamide ;
- N-[[5-(4-chlorophényl)-1-(2,4-dichlorophényl)-4-méthyl-1*H*-pyrazol-3-yl]méthyl]-butane-1-sulfonamide ;
- 3-chloro-N-[[5-(4-chlorophényl)-1-(2,4-dichlorophényl)-4-méthyl-1*H*-pyrazol-3-yl]méthyl]benzènesulfonamide ;
- 4-*tert*-butyl-N-[[5-(4-chlorophényl)-1-(2,4-dichlorophényl)-4-méthyl-1*H-*pyrazol-3-yl]méthyl]benzènesulfonamide ;
- N-[[5-(4-chlorophényl)-1-(2,4-dichlorophényl)-4-méthyl-1*H*-pyrazol-3-yl]méthyl]-3-méthoxybenzènesulfonamide ;
- N-[[5-(4-chlorophényl)-1-(2,4-dichlorophényl)-4-méthyl-1*H*-pyrazol-3-yl]méthyl]-3-cyanobenzènesulfonamide ;
- N-[[5-(4-chlorophényl)-1-(2,4-dichlorophényl)-4-méthyl-1H*-*pyrazol-3-yl]méthyl]-4-(trifluorométhyl)benzènesulfonamide ;
- N-[[5-(4-chlorophényl)-1-(2,4-dichlorophényl)-4-méthyl-1*H*-pyrazol-3-yl]méthyl]-2-(trifluorométhoxy)benzènesulfonamide ;
- N-[[5-(4-chlorophényl)-1-(2,4-dichlorophényl)-4-méthyl-1*H*-pyrazol-3-yl]méthyl]-2-(méthylsulfonyl)benzènesulfonamide ;
- 3-chloro-N-[[5-(4-chlorophényl)-1-(2,4-dichlorophényl)-4-méthyl-1*H*-pyrazol-3-yl]méthyl]-4-fluorobenzènesulfonamide ;
- 4-bromo-N-[[5-(4-chlorophényl)-1-(2,4-dichlorophényl)-4-méthyl-1*H*-pyrazol-3-yl]méthyl]-2-éthylbenzènesulfonamide ;
- N-[[5-(4-bromophényl)-1-(2,4-dichlorophényl)-4-éthyl-1*H*-pyrazol-3-yl]méthyl] éthanesulfonamide ;
- N-[[5-(4-bromophényl)-1-(2,4-dichlorophényl)-4-éthyl-1*H*-pyrazol-3-yl]méthyl] propane-2-sulfonamide ;
- N-[[5-(4-bromophényl)-1-(2,4-dichlorophényl)-4-éthyl-1*H*-pyrazol-3-yl]méthyl] butane-1-sulfonamide ;
- N-[[5-(4-bromophényl)-1-(2,4-dichlorophényl)-4-éthyl-1*H*-pyrazol-3-yl]méthyl] cyclohexanesulfonamide ;
- N-[[5-(4-bromophényl)-1-(2,4-dichlorophényl)-4-éthyl-1*H*-pyrazol-3-yl]méthyl]-1-cyclohexylméthanesulfonamide ;
- N-[[5-(4-bromophényl)-1-(2,4-dichlorophényl)-4-éthyl-1*H*-pyrazol-3-yllméthyl]-3-chlorobenzènesulfonamide ;
- N-[[5-(4-bromophényl)-1-(2,4-dichlorophényl)-4-éthyl-1*H*-pyrazol-3-yl]méthyl]-2-chlorobenzènesulfonamide ;
- N-[[5-(4-bromophényl)-1-(2,4-dichlorophényl)-4-éthyl-1*H*-pyrazol-3-yl]méthyl]-3-méthylbenzènesulfonamide ;
- N-[[5-(4-bromophényl)-1-(2,4-dichlorophényl)-4-éthyl-1*H*-pyrazol-3-yl]méthyl]-4-*tert*-butylbenzènesulfonamide ;
- N-[[5-(-4-bromophényl)-1-(2,4-dichlorophényl)-4-éthyl-1*H*-pyrazol-3 yl]méthyl]-4-méthoxybenzènesulfonamide ;
- N-[[5-(4-bromophényl)-1-(2,4-dichlorophényl)-4-éthyl-1*H*-pyrazol-3-yl]méthyl]-3-méthoxybenzènesulfonamide ;
- N-[[5-(4-bromophényl)-1-(2,4-dichlorophényl)-4-éthyl-1*H*-pyrazol-3-yl]méthyl]-4- (trifluorométhyl)benzènesulfonamide ;
- N-[[5-(4-bromophényl)-1-(2,4-dichlorophényl)-4-éthyl-1*H*-pyrazol-3-yl]méthyl]-3- (trifluorométhyl)benzènesulfonamide ;
- N-[[5-(4-bromophényl)-1-(2,4-dichlorophényl)-4-éthyl-1*H*-pyrazol-3-yl]méthyl]-2- (trifluorométhyl)benzènesulfonamide ;
- N-[[5-(4-bromophényl)-1-(2,4-dichlorophényl)-4-éthyl-1*H*-pyrazol-3-yl]méthyl]-3- (trifluorométhoxy)benzènesulfonamide ;
- N-[[5-(4-bromophényl)-1-(2,4-dichlorophényl)-4-éthyl-1*H*-pyrazol-3-yl]méthyl]-2- (trifluorométhoxy)benzènesulfonamide ;
- 3-acétyl-N-[[5-(4-bromophényl)-1-(2,4-dichlorophényl)-4-éthyl-1*H*-pyrazol-3-yl]méthyl]benzènesulfonamide ;
- N-[[5-(4-bromophényl)-1-(2,4-dichlorophényl)-4-éthyl-1*H*-pyrazol-3-yl]méthyl] biphényl-3-sulfonamide ;
- N-[[5-(4-bromophényl)-1-(2,4-dichlorophényl)-4-éthyl-1*H*-pyrazol-3-yl]méthyl]-1- [4-(trifluorométhyl)phényl]méthanesulfonamide ;
- N-[[5-(4-bromophényl)-1-(2,4-dichlorophényl)-4-éthyl-1*H*-pyrazol-3-yl]méthyl]-1- [3-(trifluorométhyl)phényl]méthanesulfonamide ;
- N-[[5-(4-bromophényl)-1-(2,4-dichlorophényl)-4-éthyl-1*H*-pyrazol-3-yl]méthyl]-3,5-diméthylbenzènesulfonamide ;
- N-[[5-(4-bromophényl)-1-(2,4-dichlorophényl)-4-éthyl-1*H*-pyrazol-3-yl]méthyl]-3,5-bis(trifluorométhyl)benzènesulfonamide ;
- 3-chloro-N-[[1-(2-chlorophényl)-5-(4-chlorophényl)-4-méthyl-1*H*-pyrazol-3-yl]méthyl]benzènesulfonamide ;
- N-[[1-(2-chlorophényl)-5-(4-chlorophényl)-4-méthyl-1*H*-pyrazol-3-yl]méthyl]-2-fluorobenzènesulfonamide ;
- N-[[1-(2-chlorophényl)-5-(4-chlorophényl)-4-méthyl-1*H*-pyrazol-3-yl]méthyl]-3-cyanobenzènesulfonamide ;
- N-[[1-(2-chlorophényl)-5-(4-chlorophényl)-4-méthyl-1*H*-pyrazol-3-yl]méthyl]-3-méthoxybenzènesulfonamide ;
- N-[[5-(4-bromophényl)-1-(2,4-dichlorophényl)-4-méthyl-1*H*-pyrazol-3-yl]méthyl]-3-méthoxybenzènesulfonamide ;
- N-[[5-(4-bromophényl)-1-(2,4-dichlorophényl)-4-méthyl-1*H*-pyrazol-3-yl]méthyl]-3-cyanobenzènesulfonamide ;
- N-[[5-(4-bromophényl)-1-(2,4-dichlorophényl)-4-éthyl-1*H*-pyrazol-3-yl]méthyl]-1-(2-fluorophényl)méthanesulfonamide ;
- N-[[5-(4-bromophényl)-1-(2,4-dichlorophényl)-4-éthyl-1*H*-pyrazol-3-yl]méthyl]-1-(4-fluorophényl)méthanesulfonamide ;
- 5-bromo-N-[[5-(4-bromophényl)-1-(2,4-dichlorophényl)-4-éthyl-1*H*-pyrazol-3-yl]méthyl]thiophène-2-sulfonamide ;
- N-[[5-(4-bromophényl)-1-(2,4-dichlorophényl)-4-éthyl-1*H*-pyrazol-3-yl]méthyl]-5-isoxazol-3-ylthiophène-2-sulfonamide ;
- 3-chloro-N-[[1-(2,4-dichlorophényl)-5-(4-méthoxyphényl)-4-méthyl-1*H-*pyrazol-3-yl]méthyl]benzènesulfonamide ;
- N-[[1-(2,4-dichlorophényl)-5-(4-méthoxyphényl)-4-méthyl-1*H*-pyrazol-3-yl]méthyl]-3-méthylbenzènesulfonamide ;
à l'état de base ou de sel d'addition à un acide, ainsi qu'à l'état d'hydrate ou de solvat.

4. Procédé de préparation des composés de formule (I) selon la revendication 1, **caractérisé en ce que :**
on fait réagir, en présence d'une base et dans un solvant, un composé de formule : 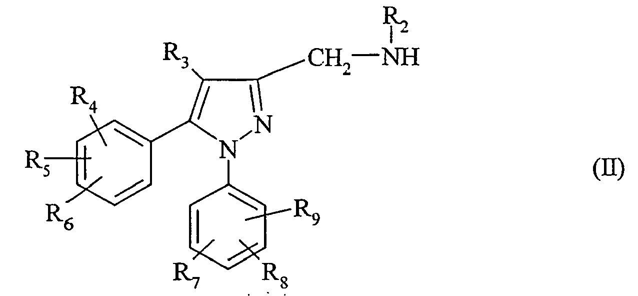
dans laquelle R₂, R₃, R₄, R₅, R₆, R₇, R₈ et R₉ sont tels que définis pour un composé de formule (I) dans la revendication 1, avec un halogénure de sulfonyle de formule : 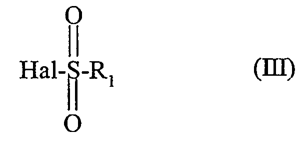 dans laquelle R₁ est tel que défini pour un composé de formule (I) dans la revendication 1 et Hal représente un atome d'halogène.

5. Médicament, **caractérisé en ce qu'**il comprend un composé de formule (I) selon l'une quelconque des revendications 1 à 3, ou un sel d'addition de ce composé à un acide pharmaceutiquement acceptable, ou encore un hydrate ou un solvat du composé de formule (I).

6. Composition pharmaceutique, **caractérisée en ce qu'**elle comprend un composé de formule (I) selon l'une quelconque des revendications 1 à 3, ou un sel pharmaceutiquement acceptable, un hydrate ou un solvat de ce composé, ainsi qu'au moins un excipient pharmaceutiquement acceptable.

7. Utilisation d'un composé de formule (I) selon l'une quelconque des revendications 1 à 3 pour la préparation d'un médicament destiné au traitement et à la prévention des troubles de l'appétit, des troubles gastro-intestinaux, des phénomènes inflammatoires, des maladies du système immunitaire, des troubles psychotiques, de la dépendance alcoolique, de la dépendance nicotinique.

## Claims

1. Compound corresponding to formula (I): 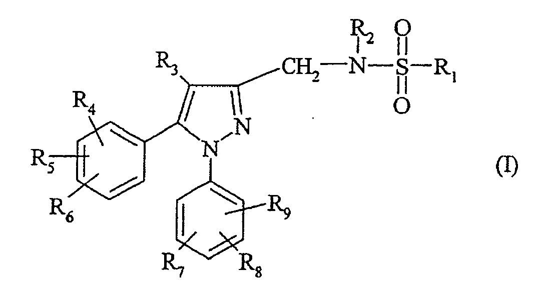 in which:
- R₁ represents
• a (C₁-C₆) alkyl;
• a (C₃-C₇)cycloalkyl which is unsubstituted or substituted once or several times with a (C1-C3)alkyl group;
• a (C₃-C₇)cycloalkylmethyl which is unsubstituted or substituted once or several times on the carbocycle with a (C₁-C₃)alkyl;
• a phenyl which is unsubstituted or mono-, di- or trisubstituted with a substituent independently chosen from a halogen atom, a (C₁-C₄)alkyl, a (C₁-C₃)alkoxy, a cyano, a trifluoromethyl radical, a trifluoromethoxy radical, an S(O)ₙAlk group, a (C₁-C₃) alkylcarbonyl group, a phenyl;
• a benzyl which is unsubstituted or mono- or disubstituted with a substituent independently chosen from a halogen atom, a (C₁-C₃)alkyl, a (C₁-C₃)alkoxy; a trifluoromethyl radical;
• a thienyl which is unsubstituted or substituted with a halogen atom or with an isoxazolyl;
- R₂ represents a hydrogen atom or a (C₁-C₃)alkyl;
- R₃ represents a hydrogen atom or a (C₁-C₅)alkyl;
- R₄, R₅, R₆, R₇, R₈ and R₉ each independently represent a hydrogen atom, a halogen atom, a (C₁-C₇)alkyl, a (C₁-C₅)alkoxy, a trifluoromethyl radical or an S(O)ₙAlk group;
- n represents 0, 1 or 2;
- Alk represents a (C₁-C₄)alkyl,
in the form of a base or an addition salt with an acid, and in the form of a hydrate or a solvate.

2. Compound of formula (I) according to Claim 1, in which:
- R₁ represents
• an ethyl, an isopropyl, an n-butyl;
• a cyclohexyl;
• a cyclohexylmethyl;
• a 2-chlorophenyl, a 3-chlorophenyl, a 2-fluorophenyl, a 3-chloro-4-fluorophenyl, a 4-bromo-2-ethylphenyl, a 3-methylphenyl, a 4-*tert*-butylphenyl, a 3,5-dimethylphenyl, a 3-methoxyphenyl, a 4-methoxyphenyl, a 3-cyanophenyl, a 4-cyanophenyl, a 2-(trifluoromethyl)phenyl, a 3-(trifluoromethyl)phenyl, a 4-(trifluoromethyl)phenyl, a 3,5-bis(trifluoromethyl)phenyl, a 2-(trifluoromethoxy)phenyl, a 3-(trifluoromethoxy)phenyl, a 2-(methylsulfonyl)-phenyl, a 3-(methylsulfonyl)phenyl, a 3-acetylphenyl, a 3-biphenyl, a 2-biphenyl;
• a 3-chlorobenzyl, a 2-fluorobenzyl, a 4-fluorobenzyl, a 3-(trifluoromethyl)benzyl, a 4-(trifluoromethyl)benzyl;
• a 5-bromo-2-thienyl; a 5-isoxazol-3-yl-2-thienyl;
- R₂ represents a hydrogen atom or a methyl;
- R₃ represents a methyl or an ethyl;
- R₄ represents a hydrogen atom;
- R₅ is at the 4-position of the phenyl and represents a bromine, chlorine or fluorine atom, or a methoxy;
- R₆ represents a hydrogen atom;
- R₇ represents a hydrogen atom;
- R₈ is at the 4-position of the phenyl and represents a hydrogen atom, a chlorine atom, a fluorine atom;
- R₉ is at the 2-position of the phenyl and represents a chlorine or fluorine atom;
in the form of a base or of an addition salt with an acid, and in the form of a hydrate or a solvate.

3. Compound of formula (I) according to Claim 1, chosen from:
- N-[[5-(4-bromophenyl)-1-(2,4-dichlorophenyl)-4-methyl-1*H*-pyrazol-3-yl]methyl]butane-1-sulfonamide;
- N-[[5-(4-bromophenyl)-1-(2,4-dichlorophenyl)-4-methyl-1*H*-pyrazol-3-yl]methyl]-cyclohexanesulfonamide;
- N-[[5-(4-bromophenyl)-1-(2,4-dichlorophenyl)-4-methyl-1*H*-pyrazol-3-yl]methyl]-cyclohexylmethanesulfonamide;
- N-[[5-(4-bromophenyl)-1-(2,4-dichlorophenyl)-4-methyl-1*H*-pyrazol-3-yl]methyl]-3-chlorobenzenesulfonamide;
- N-[[5-(4-bromophenyl)-1-(2,4-dichlorophenyl)-4-methyl-1*H*-pyrazol-3-yl]methyl]-4-*tert-*butylbenzenesulfonamide;
- N-[[5-(4-bromophenyl)-1-(2,4-dichlorophenyl)-4-methyl-1*H*-pyrazol-3-yl]methyl]-3-methoxybenzenesulfonamide;
- N-[[5-(4-bromophenyl)-1-(2,4-dichlorophenyl)-4-methyl-1*H*-pyrazol-3-yl]methyl]-4-methoxybenzenesulfonamide;
- N-[[5-(4-bromophenyl)-1-(2,4-dichlorophenyl)-4-methyl-1*H*-pyrazol-3-yl]methyl]-4-(trifluoromethyl)benzenesulfonamide;
- N-[[5-(4-bromophenyl)-1-(2,4-dichlorophenyl)-4-methyl-1*H*-pyrazol-3-yl]methyl]-2-(methylsulfonyl)benzenesulfonamide;
- N-[[5-(4-bromophenyl)-1-(2,4-dichlorophenyl)-4-methyl-1*H*-pyrazol-3-yl]methyl]-1-(3-chlorophenyl)methanesulfonamide;
- N-[[5-(4-bromophenyl)-1-(2,4-dichlorophenyl)-4-methyl-1*H*-pyrazol-3-yl]methyl]-1-[3-(trifluoromethyl)phenyl]methanesulfonamide;
- N-[[5-(4-bromophenyl)-1-(2,4-dichlorophenyl)-4-methyl-1*H*-pyrazol-3-yl]methyl]-3-chloro-4-fluorobenzenesulfonamide;
- N-[[5-(4-chlorophenyl)-1-(2,4-dichlorophenyl)-4-methyl-1*H*-pyrazol-3-yl]methyl]-butane-1-sulfonamide;
- 3-chloro-N-[[5-(4-chlorophenyl)-1-(2,4-dichlorophenyl)-4-methyl-1*H*-pyrazol-3-yl]methyl]benzenesulfonamide;
- 4-*tert*-butyl-N-[[5-(4-chlorophenyl)-1-(2,4-dichlorophenyl)-4-methyl-1*H*-pyrazol-3-yl]methyl]benzenesulfonamide;
- N-[[5-(4-chlorophenyl)-1-(2,4-dichlorophenyl)-4-methyl-1*H*-pyrazol-3-yl]methyl]-3-methoxybenzenesulfonamide;
- N-[[5-(4-chlorophenyl)-1-(2,4-dichlorophenyl)-4-methyl-1*H*-pyrazol-3-yl]methyl]-3-cyanobenzenesulfonamide;
- N-[[5-(4-chlorophenyl)-1-(2,4-dichlorophenyl)-4-methyl-1*H*-pyrazol-3-yl]methyl]-4-(trifluoromethyl)benzenesulfonamide;
- N-[[5-(4-chlorophenyl)-1-(2,4-dichlorophenyl)-4-methyl-1*H*-pyrazol-3-yl]methyl]-2-(trifluoromethoxy)benzenesulfonamide;
- N-[[5-(4-chlorophenyl)-1-(2,4-dichlorophenyl)-4-methyl-1*H*-pyrazol-3-yl]methyl]-2-(methylsulfonyl)benzenesulfonamide;
- 3-chloro-N-[[5-(4-chlorophenyl)-1-(2,4-dichlorophenyl)-4-methyl-1*H*-pyrazol-3-yl]methyl]-4-fluorobenzenesulfonamide;
- 4-bromo-N-[[5-(4-chlorophenyl)-1-(2,4-dichlorophenyl)-4-methyl-1*H*-pyrazol-3-yl]methyl]-2-ethylbenzenesulfonamide;
- N-[[5-(4-bromophenyl)-1-(2,4-dichlorophenyl)-4-ethyl-1*H*-pyrazol-3-yl]methyl]ethanesulfonamide;
- N-[[5-(4-bromophenyl)-1-(2,4-dichlorophenyl)-4-ethyl-1*H*-pyrazol-3-yl]methyl]propane-2-sulfonamide;
- N-[[5-(4-bromophenyl)-1-(2,4-dichlorophenyl)-4-ethyl-1*H*-pyrazol-3-yl]methyl]butane-1-sulfonamide;
- N-[[5-(4-bromophenyl)-1-(2,4-dichlorophenyl)-4-ethyl-1*H*-pyrazol-3-yl]methyl]-cyclohexanesulfonamide;
- N-[[5-(4-bromophenyl)-1-(2,4-dichlorophenyl)-4-ethyl-1*H*-pyrazol-3-yl]methyl]-1-cyclohexylmethanesulfonamide;
- N-[[5-(4-bromophenyl)-1-(2,4-dichlorophenyl)-4-ethyl-1*H*-pyrazol-3-yl]methyl]-3-chlorobenzenesulfonamide;
- N-[[5-(4-bromophenyl)-1-(2,4-dichlorophenyl)-4-ethyl-1*H*-pyrazol-3-yl]methyl]-2-chlorobenzenesulfonamide;
- N-[[5-(4-bromophenyl)-1-(2,4-dichlorophenyl)-4-ethyl-1*H*-pyrazol-3-yl]methyl]-3-methylbenzenesulfonamide;
- N-[[5-(4-bromophenyl)-1-(2,4-dichlorophenyl)-4-ethyl-1*H*-pyrazol-3-yl]methyl]-4-*tert-*butylbenzenesulfonamide;
- N-[[5-(4-bromophenyl)-1-(2,4-dichlorophenyl)-4-ethyl-1*H*-pyrazol-3-yl]methyl]-4-methoxybenzenesulfonamide;
- N-[[5-(4-bromophenyl)-1-(2,4-dichlorophenyl)-4-ethyl-1*H*-pyrazol-3-yl]methyl]-3-methoxybenzenesulfonamide;
- N-[[5-(4-bromophenyl)-1-(2,4-dichlorophenyl)-4-ethyl-1*H*-pyrazol-3-yl]methyl]-4-(trifluoromethyl)benzenesulfonamide;
- N-[[5-(4-bromophenyl)-1-(2,4-dichlorophenyl)-4-ethyl-1*H*-pyrazol-3-yl]methyl]-3-(trifluoromethyl)benzenesulfonamide;
- N-[[5-(4-bromophenyl)-1-(2,4-dichlorophenyl)-4-ethyl-1*H*-pyrazol-3-yl]methyl]-2-(trifluoromethyl)benzenesulfonamide;
- N-[[5-(4-bromophenyl)-1-(2,4-dichlorophenyl)-4-ethyl-1*H*-pyrazol-3-yl]methyl]-3-(trifluoromethoxy)benzenesulfonamide;
- N-[[5-(4-bromophenyl)-1-(2,4-dichlorophenyl)-4-ethyl-1*H*-pyrazol-3-yl]methyl]-2-(trifluoromethoxy)benzenesulfonamide;
- 3-acetyl-N-[[5-(4-bromophenyl)-1-(2,4-dichlorophenyl)-4-ethyl-1*H*-pyrazol-3-yl]methyl]benzenesulfonamide;
- N-[[5-(4-bromophenyl)-1-(2,4-dichlorophenyl)-4-ethyl-1*H*-pyrazol-3-yl]methyl]biphenyl-3-sulfonamide;
- N-[[5-(4-bromophenyl)-1-(2,4-dichlorophenyl)-4-ethyl-1*H*-pyrazol-3-yl]methyl]-1-[4-(trifluoromethyl)phenyl]methanesulfonamide;
- N-[[5-(4-bromophenyl)-1-(2,4-dichlorophenyl)-4-ethyl-1*H*-pyrazol-3-yl]methyl]-1-[3-(trifluoromethyl)phenyl]methanesulfonamide;
- N-[[5-(4-bromophenyl)-1-(2,4-dichlorophenyl)-4-ethyl-1*H*-pyrazol-3-yl]methyl]-3,5-dimethylbenzenesulfonamide;
- N-[[5-(4-bromophenyl)-1-(2,4-dichlorophenyl)-4-ethyl-1*H*-pyrazol-3-yl]methyl]-3,5-bis(trifluoromethyl)benzenesulfonamide;
- 3-chloro-N-[[1-(2-chlorophenyl)-5-(4-chlorophenyl)-4-methyl-1*H*-pyrazol-3-yl]methyl]benzenesulfonamide;
- N-[[1-(2-chlorophenyl)-5-(4-chlorophenyl)-4-methyl-1*H*-pyrazol-3-yl]methyl]-2-fluorobenzenesulfonamide;
- N-[[1-(2-chlorophenyl)-5-(4-chlorophenyl)-4-methyl-1*H*-pyrazol-3-yl]methyl]-3-cyanobenzenesulfonamide;
- N-[[1-(2-chlorophenyl)-5-(4-chlorophenyl)-4-methyl-1*H*-pyrazol-3-yl]methyl]-3-methoxybenzenesulfonamide;
- N-[[5-(4-bromophenyl)-1-(2,4-dichlorophenyl)-4-methyl-1*H*-pyrazol-3-yl]methyl]-3-methoxybenzenesulfonamide;
- N-[[5-(4-bromophenyl)-1-(2,4-dichlorophenyl)-4-methyl-1*H*-pyrazol-3-yl]methyl]-3-cyanobenzenesulfonamide;
- N-[[5-(4-bromophenyl)-1-(2,4-dichlorophenyl)-4-ethyl-1*H*-pyrazol-3-yl]methyl]-1-(2-fluorophenyl)methanesulfonamide;
- N-[[5-(4-bromophenyl)-1-(2,4-dichlorophenyl)-4-ethyl-1*H*-pyrazol-3-yl]methyl]-1-(4-fluorophenyl)methanesulfonamide;
- 5-bromo-N-[[5-(4-bromophenyl)-1-(2,4-dichlorophenyl)-4-ethyl-1H-pyrazol-3-yl]methyl]thiophene-2-sulfonamide;
- N-[[5-(4-bromophenyl)-1-(2,4-dichlorophenyl)-4-ethyl-1*H*-pyrazol-3-yl]methyl]-5-isoxazol-3-ylthiophene-2-sulfonamide;
- 3-chloro-N-[[1-(2,4-dichlorophenyl)-5-(4-methoxyphenyl)-4-methyl-1*H*-pyrazol-3-yl]methyl]benzenesulfonamide;
- N-[[1-(2,4-dichlorophenyl)-5-(4-methoxyphenyl)-4-methyl-1*H*-pyrazol-3-yl]methyl]-3-methylbenzenesulfonamide;
in the form of a base or of an addition salt with an acid, and in the form of a hydrate or of a solvate.

4. Method for preparing the compounds of formula (I) according to Claim 1, **characterized in that**:
a compound of formula: 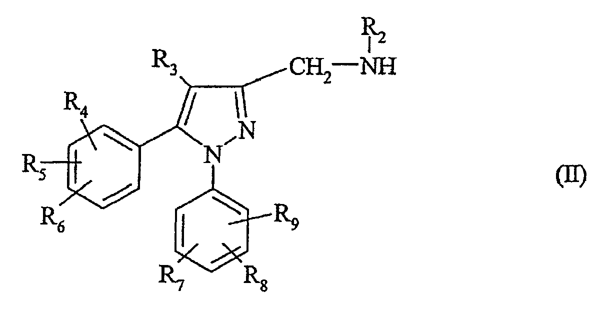 in which R₂, R₃, R₄, R₅, R₆, R₇, R₈ and R₉ are as defined for a compound of formula (I) in Claim 1, is reacted, in the presence of a base and in a solvent, with a sulfonyl halide of formula: 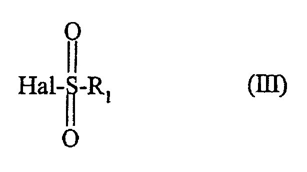 in which R₁ is as defined for a compound of formula (I) in Claim 1 and Hal represents a halogen atom.

5. Medicament, **characterized in that** it comprises a compound of formula (I) according to any one of Claims 1 to 3, or an addition salt of this compound with a pharmaceutically acceptable acid, or a hydrate or a solvate of the compound of formula (I).

6. Pharmaceutical composition, **characterized in that** it comprises a compound of formula (I) according to any one of Claims 1 to 3, or a pharmaceutically acceptable salt, a hydrate or a solvate of this compound, and at least one pharmaceutically acceptable excipient.

7. Use of a compound of formula (I) according to any one of Claims 1 to 3 for the preparation of a medicament intended for the treatment and prevention of appetite disorders, gastrointestinal disorders, inflammatory phenomena, immune system diseases, psychotic disorders, alcohol dependence and nicotine dependence.

## Patentansprüche

1. Verbindung der Formel (I): 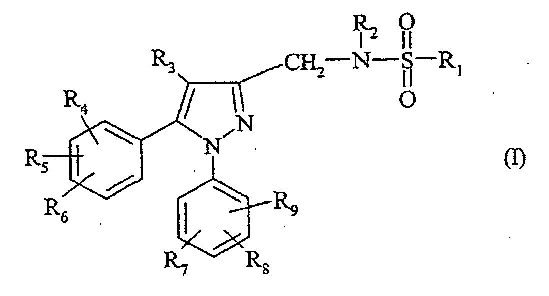 worin:
R₁ für
. (C₁-C₆) -Alkyl;
. (C₃-C₇)-Cycloalkyl, das gegebenenfalls ein- oder mehrfach durch eine (C₁-C₃)-Alkylgruppe substituiert ist;
. (C₃-C₇)-Cycloalkylmethyl, das gegebenenfalls am Carbocyclus ein- oder mehrfach durch eine (C₁-C₃) - Alkylgruppe substituiert ist;
. Phenyl, das gegebenenfalls ein-, zwei- oder dreifach durch einen unabhängig voneinander unter einem Halogenatom, (C₁-C₄) -Alkyl, (C₁-C₃) -Alkoxy, Cyano, einem Trifluormethylrest, einem Trifluormethoxyrest, einer S(O)ₙAlk-Gruppe, einer (C₁-C₃) - Alkylcarbonylgruppe und Phenyl ausgewählten Substituenten substituiert ist;
. Benzyl, das gegebenenfalls ein- oder zweifach durch einen unabhängig voneinander unter einem Halogenatom, (C₁-C₃) -Alkyl, (C₁-C₃)-Alkoxy und einem Trifluormethylrest ausgewählten Substituenten substituiert ist;
. Thienyl, das gegebenenfalls durch ein Halogenatom oder Isoxazolyl substituiert ist;
steht;
- R₂ für ein Wasserstoffatom oder (C₁-C₃)-Alkyl steht;
- R₃ für ein Wasserstoffatom oder (C₁-C₅)-Alkyl steht;
- R₄, R₅, R₆, R₇, R₈ und R₉ jeweils unabhängig voneinander für ein Wasserstoffatom, ein Halogenatom, (C₁-C₇)-Alkyl, (C₁-C₅)-Alkoxy, einen Trifluormethylrest oder eine S(O)ₙAlk-Gruppe stehen;
- n für 0, 1 oder 2 steht;
- Alk für (C₁-C₄)-Alkyl steht;
in Basen- oder Säureadditionssalzform sowie in Hydrat- oder Solvatform.

2. Verbindung der Formel (I) nach Anspruch 1, worin:
- R₁ für
. Ethyl, Isopropyl oder n-Butyl;
. Cyclohexyl;
. Cyclohexylmethyl;
. 2-Chlorphenyl, 3-Chlorphenyl, 2-Fluorphenyl, 3-Chlor-4-fluorphenyl, 4-Brom-2-ethylphenyl, 3-Methylphenyl, 4-*tert*-Butylphenyl, 3,5-Dimethylphenyl, 3-Methoxyphenyl, 4-Methoxyphenyl, 3-Cyanophenyl, 4-Cyanophenyl, 2-(Trifluormethyl)phenyl, 3-(Trifluormethyl)phenyl, 4-(Trifluormethyl)-phenyl, 3,5-Bis(trifluormethyl)phenyl, 2-(Trifluormethoxy)phenyl, 3-(Trifluormethoxy)phenyl, 2-(Methylsulfonyl)phenyl, 3-(Methylsulfonyl)phenyl, 3-Acetylphenyl, 3-Biphenyl oder 2-Biphenyl;
. 3-Chlorbenzyl, 2-Fluorbenzyl, 4-Fluorbenzyl, 3-(Trifluormethyl)benzyl oder 4-(Trifluormethyl)-benzyl;
. 5-Brom-2-thienyl oder 5-Isoxazaol-3-yl-2-thienyl steht;
- R₂ für ein Wasserstoffatom oder Methyl steht;
- R₃ für Methyl oder Ethyl steht;
- R₄ für ein Wasserstoffatom steht;
- R₅ in der 4-Position des Phenyls steht und für ein Brom-, Chlor- oder Fluoratom oder Methoxy steht;
- R₆ für ein Wasserstoffatom steht;
- R₇ für ein Wasserstoffatom steht;
- R₈ in der 4-Position des Phenyls steht und für ein Wasserstoffatom, ein Chloratom oder ein Fluoratom steht;
- R₉ in der 2-Position des Phenyls steht und für ein Chlor- oder Fluoratom steht;
in Basen- oder Säureadditionssalzform sowie in Hydrat- oder Solvatform.

3. Verbindung der Formel (I) nach Anspruch 1, ausgewählt unter:
- N-[[5-(4-Bromphenyl)-1-(2,4-dichlorphenyl)-4-methyl-1*H*-pyrazol-3-yl]methyl]butan-1-sulfonamid;
- N-[[5-(4-Bromphenyl)-1-(2,4-dichlorphenyl)-4-methyl-1*H*-pyrazol-3-yl]methyl]-cyclohexansulfonamid;
- N-[[5-(4-Bromphenyl)-1-(2,4-dichlorphenyl)-4-methyl-1*H*-pyrazol-3-yl]methyl]-cyclohexylmethansulfonamid;
- N-[[5-(4-Bromphenyl)-1-(2,4-dichlorphenyl)-4-methyl-1*H*-pyrazol-3-yl]methyl]-3-chlorbenzolsulfonamid;
- N-[[5-(4-Bromphenyl)-1-(2,4-dichlorphenyl)-4-methyl-1*H*-pyrazol-3-yl]methyl]-4-*tert*-butylbenzolsulfonamid;
- N-[[5-(4-Bromphenyl)-1-(2,4-dichlorphenyl)-4-methyl-1*H*-pyrazol-3-yl]methyl]-3-methoxybenzolsulfonamid;
- N-[[5-(4-Bromphenyl)-1-(2,4-dichlorphenyl)-4-methyl-1*H*-pyrazol-3-yl]methyl]-4-methoxybenzolsulfonamid;
- N-[[5-(4-Bromphenyl)-1-(2,4-dichlorphenyl)-4-methyl-1*H*-pyrazol-3-yl]methyl]-4-(trifluormethyl)-benzolsulfonamid;
- N-[[5-(4-Bromphenyl)-1-(2,4-dichlorphenyl)-4-methyl-1*H*-pyrazol-3-yl]methyl]-2-(methylsulfonyl)-benzolsulfonamid;
- N-[[5-(4-Bromphenyl)-1-(2,4-dichlorphenyl)-4-methyl-1*H*-pyrazol-3-yl]methyl]-1-(3-chlorphenyl)-methansulfonamid;
- N-[[5-(4-Bromphenyl)-1-(2,4-dichlorphenyl)-4-methyl-1*H*-pyrazol-3-yl]methyl]-1-[3-(trifluormethyl)phenyl]methansulfonamid;
- N-[[5-(4-Bromphenyl)-1-(2,4-dichlorphenyl)-4-methyl-1*H*-pyrazol-3-yl]methyl]-3-chlor-4-fluorbenzolsulfonamid;
- N-[[5-(4-Chlorphenyl)-1-(2,4-dichlorphenyl)-4-methyl-1*H*-pyrazol-3-yl]methyl]-butan-1-sulfonamid;
- 3-chlor-N-[[5-(4-chlorphenyl)-1-(2,4-dichlorphenyl)-4-methyl-1*H*-pyrazol-3-yl]methyl]benzolsulfonamid;
- 4-*tert*-Butyl-N-[[5-(4-chlorphenyl)-1-(2,4-dichlorphenyl)-4-methyl-1*H*-pyrazol-3-yl]methyl]-benzolsulfonamid;
- N-[[5-(4-Chlorphenyl)-1-(2,4-dichlorphenyl)-4-methyl-1*H*-pyrazol-3-yl]methyl]-3-methoxybenzolsulfonamid;
- N-[[5-(4-Chlorphenyl)-1-(2,4-dichlorphenyl)-4-methyl-1*H*-pyrazol-3-yl]methyl]-3-cyanobenzolsulfonamid;
- N-[[5-(4-Chlorphenyl)-1-(2,4-dichlorphenyl)-4-methyl-1*H*-pyrazol-3-yl]methyl]-4-(trifluormethyl)-benzolsulfonamid;
- N-[[5-(4-Chlorphenyl)-1-(2,4-dichlorphenyl)-4-methyl-1*H*-pyrazol-3-yl]methyl]-2-(trifluormethoxy)benzolsulfonamid;
- N-[[5-(4-Chlorphenyl)-1-(2,4-dichlorphenyl)-4-methyl-1*H*-pyrazol-3-yl]methyl]-2-(methylsulfonyl)-benzolsulfonamid;
- 3-Chlor-N-[[5-(4-chlorphenyl)-1-(2,4-dichlorphenyl)-4-methyl-1*H*-pyrazol-3-yl]methyl]-4-fluorbenzolsulfonamid;
- 4-Brom-N-[[5-(4-chlorphenyl)-1-(2,4-dichlorphenyl)-4-methyl-1*H*-pyrazol-3-yl]methyl]-2-ethylbenzolsulfonamid;
- N-[[5-(4-Bromphenyl)-1-(2,4-dichlorphenyl)-4-ethyl-1*H*-pyrazol-3-yl]methyl]ethansulfonamid;
- N-[[5-(4-Bromphenyl)-1-(2,4-dichlorphenyl)-4-ethyl-1*H*-pyrazol-3-yl]methyl]propan-2-sulfonamid;
- N-[[5-(4-Bromphenyl)-1-(2,4-dichlorphenyl)-4-ethyl-1*H*-pyrazol-3-yl]methyl]butan-1-sulfonamid;
- N-[[5-(4-Bromphenyl)-1-(2,4-dichlorphenyl)-4-ethyl-1*H*-pyrazol-3-yl]methyl]-cyclohexansulfonamid;
- N-[[5-(4-Bromphenyl)-1-(2,4-dichlorphenyl)-4-ethyl-1*H*-pyrazol-3-yl]methyl]-1-cyclohexylmethansulfonamid;
- N-[[5-(4-Bromphenyl)-1-(2,4-dichlorphenyl)-4-ethyl-1*H*-pyrazol-3-yl]methyl]-3-chlorbenzolsulfonamid;
- N-[[5-(4-Bromphenyl)-1-(2,4-dichlorphenyl)-4-ethyl-1*H*-pyrazol-3-yl]methyl]-2-chlorbenzolsulfonamid;
- N-[[5-(4-Bromphenyl)-1-(2,4-dichlorphenyl)-4-ethyl-1*H*-pyrazol-3-yl]methyl]-3-methylbenzolsulfonamid;
- N-[[5-(4-Bromphenyl)-1-(2,4-dichlorphenyl)-4-ethyl-1*H*-pyrazol-3-yl]methyl]-4-*tert*-butylbenzolsulfonamid;
- N-[[5-(4-Bromphenyl)-1-(2,4-dichlorphenyl)-4-ethyl-1*H*-pyrazol-3-yl]methyl]-4-methoxybenzolsulfonamid;
- N-[[5-(4-Bromphenyl)-1-(2,4-dichlorphenyl)-4-ethyl-1*H*-pyrazol-3-yl]methyl]-3-methoxybenzolsulfonamid;
- N-[[5-(4-Bromphenyl)-1-(2,4-dichlorphenyl)-4-ethyl-1*H*-pyrazol-3-yl]methyl]-4-(trifluormethyl)-benzolsulfonamid;
- N-[[5-(4-Bromphenyl)-1-(2,4-dichlorphenyl)-4-ethyl-1*H*-pyrazol-3-yl]methyl]-3-(trifluormethyl)-benzolsulfonamid;
- N-[[5-(4-Bromphenyl)-1-(2,4-dichlorphenyl)-4-ethyl-1*H*-pyrazol-3-yl]methyl]-2-(trifluormethyl)-benzolsulfonamid;
- N-[[5-(4-Bromphenyl)-1-(2,4-dichlorphenyl)-4-ethyl-1*H*-pyrazol-3-yl]methyl]-3-(trifluormethoxy)-benzolsulfonamid;
- N-[[5-(4-Bromphenyl)-1-(2,4-dichlorphenyl)-4-ethyl-1*H*-pyrazol-3-yl]methyl]-2-(trifluormethoxy)-benzolsulfonamid;
- 3-Acetyl-N-[[5-(4-bromphenyl)-1-(2,4-dichlorphenyl)-4-ethyl-1*H*-pyrazol-3-yl]methyl]benzolsulfonamid;
- N-[[5-(4-Bromphenyl)-1-(2,4-dichlorphenyl)-4-ethyl-1*H*-pyrazol-3-yl]methyl]biphenyl-3-sulfonamid;
- N-[[5-(4-Bromphenyl)-1-(2,4-dichlorphenyl)-4-ethyl-1*H*-pyrazol-3-yl]methyl]-1-[4-(trifluormethyl)phenyl]methansulfonamid;
- N-[[5-(4-Bromphenyl)-1-(2,4-dichlorphenyl)-4-ethyl-1*H*-pyrazol-3-yl]methyl]-1-[3-(trifluormethyl)phenyl]methansulfonamid;
- N-[[5-(4-Bromphenyl)-1-(2,4-dichlorphenyl)-4-ethyl-1*H*-pyrazol-3-yl]methyl]-3,5-dimethylbenzolsulfonamid;
- N-[[5-(4-Bromphenyl)-1-(2,4-dichlorphenyl)-4-ethyl-1*H*-pyrazol-3-yl]methyl]-3,5-bis(trifluormethyl)benzolsulfonamid;
- 3-Chlor-N-[[1-(2-chlorphenyl)-5-(4-chlorphenyl)-4-methyl-1*H*-pyrazol-3-yl]methyl]benzolsulfonamid;
- N-[[1-(2-Chlorphenyl)-5-(4-chlorphenyl)-4-methyl-1*H*-pyrazol-3-yl]methyl]-2-fluorbenzolsulfonamid;
- N-[[1-(2-Chlorphenyl)-5-(4-chlorphenyl)-4-methyl-1*H*-pyrazol-3-yl]methyl]-3-cyanobenzolsulfonamid;
- N-[[1-(2-Chlorphenyl)-5-(4-chlorphenyl)-4-methyl-1*H*-pyrazol-3-yl]methyl]-3-methoxybenzolsulfonamid;
- N-[[5-(4-Bromphenyl)-1-(2,4-dichlorphenyl)-4-methyl-1*H*-pyrazol-3-yl]methyl]-3-methoxybenzolsulfonamid;
- N-[[5-(4-Bromphenyl)-1-(2,4-dichlorphenyl)-4-methyl-1*H*-pyrazol-3-yl]methyl]-3-cyanobenzolsulfonamid;
- N-[[5-(4-Bromphenyl)-1-(2,4-dichlorphenyl)-4-ethyl-1*H*-pyrazol-3-yl]methyl]-1-(2-fluorphenyl)-methansulfonamid;
- N-[[5-(4-Bromphenyl)-1-(2,4-dichlorphenyl)-4-ethyl-1*H*-pyrazol-3-yl]methyl]-1-(4-fluorphenyl)-methansulfonamid;
- 5-Brom-N-[[5-(4-bromphenyl)-1-(2,4-dichlorphenyl)-4-ethyl-1*H*-pyrazol-3-yl]methyl]thiophen-2-sulfonamid;
- N-[[5-(4-Bromphenyl)-1-(2,4-dichlorphenyl)-4-ethyl-1*H*-pyrazol-3-yl]methyl]-5-isoxazol-3-yl-thiophen-2-sulfonamid;
- 3-Chlor-N-[[1-(2,4-dichlorphenyl)-5-(4-methoxyphenyl)-4-methyl-1*H*-pyrazol-3-yl]methyl]-benzolsulfonamid;
- N-[[1-(2,4-Dichlorphenyl)-5-(4-methoxyphenyl)-4-methyl-1*H*-pyrazol-3-yl]methyl]-3-methylbenzolsulfonamid;
in Basen- oder Säureadditionssalzform sowie in Hydrat- oder Solvatform.

4. Verfahren zur Herstellung einer Verbindung der Formel (I) nach Anspruch 1, **dadurch gekennzeichnet, daß** man eine Verbindung der Formel: 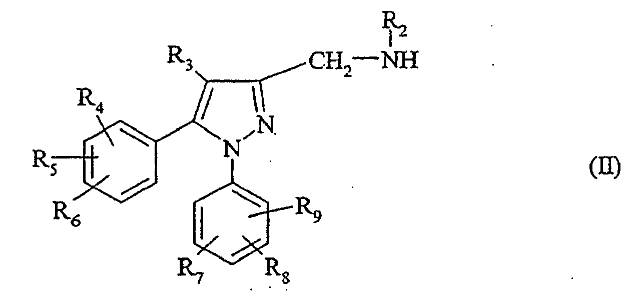 Worin R₂, R₃, R₄, R₅, R₆, R₇, R₈ und R₉, wie für eine Verbindung der Formel (I) in Anspruch 1 definiert sind, in Gegenwart einer Base und in einem Lösungsmittel mit einem Sulfonylhalogenid der Formel: 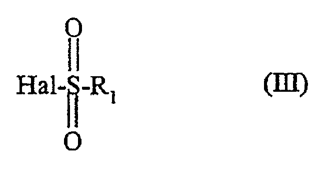 worin R₁ wie für eine Verbindung der Formel (I) in Anspruch 1 definiert ist und Hal für ein Halogenatom steht, umsetzt.

5. Arzneimittel, **dadurch gekennzeichnet, daß** es eine Verbindung der Formel (I) nach einem der Ansprüche 1 bis 3 oder ein Additionssalz dieser Verbindung mit einer pharmazeutisch unbedenklichen Säure oder auch ein Hydrat oder ein Solvat einer Verbindung der Formel (I) enthält.

6. Pharmazeutische Zusammensetzung, **dadurch gekennzeichnet, daß** sie eine Verbindung der Formel (I) nach einem der Ansprüche 1 bis 3 oder ein pharmazeutisch unbedenkliches Salz, ein Hydrat oder ein Solvat dieser Verbindung sowie einen pharmazeutisch unbedenklichen Hilfsstoff enthält.

7. Verwendung einer Verbindung der Formel (I) nach den Ansprüchen 1 bis 3 zur Herstellung eines Arzneimittels zur Behandlung oder Prävention von Appetitstörungen, Magen-Darm-Störungen, Entzündungsphänomenen, Erkrankungen des Immunsystems, psychotischen Störungen, Alkoholabhängigkeit und Nikotinabhängigkeit.
